# EUROPÄISCHE PATENTSCHRIFT

(11) **EP 1 522 343 B1**
(45) Veröffentlichungstag und Bekanntmachung des Hinweises auf die Patenterteilung: **31.07.2013**
(21) Anmeldenummer: 04023724.0
(22) Anmeldetag: 05.10.2004
(51) Int. Cl.: B01L 3/00, G01N 31/22, G01N 33/48

(54) **Analytisches Testelement umfassend ein hydrophiles Netzwerk zur Bildung eines Kapillarkanals, dessen Verwendung und Verfahren zur Bestimmung eines Analyten in einer Flüssigkeit**
Analytical testelement including a hydrophilic network for forming a capillary channel, its use and method for determining an analyte in a liquid.
Dispositif de test analytique comprenant une matrice hydrophile pour former un canal capillaire, son utilisation et procédé pour déterminer un analyte dans un liquide.

(30) Priorität: 07.10.2003 DE 10346417
(43) Veröffentlichungstag der Anmeldung: 13.04.2005
(73) Patentinhaber: Roche Diagnostics GmbH, 68305 Mannheim (DE); F.Hoffmann-La Roche AG, 4070 Basel (CH)
(72) Erfinder: Brauner, Michael, 64653 Lorsch (DE)

(56) Entgegenhaltungen:
- DE-A1- 19 753 847
- US-A- 4 323 536
- US-A- 5 104 811
- US-B1- 6 592 815

## Beschreibung

Die Anmeldung betrifft ein analytisches Testelement zur Bestimmung mindestens eines Analyten in einer Flüssigkeit, die Verwendung eines erfindungsgemäßen analytischen Testelements zur Bestimmung eines Analyten in einer Flüssigkeit sowie Verfahren zur Bestimmung eines Analyten in einer Flüssigkeit mit Hilfe eines erfindungsgemäßen analytischen Testelements.

### Stand der Technik

Zur qualitativen oder quantitativen analytischen Bestimmung von Bestandteilen von Flüssigkeiten, insbesondere von Körperflüssigkeiten wie Blut, werden oft sogenannte trägergebundene Tests verwendet. Bei diesen sind Reagenzien, insbesondere spezifische Nachweisreagenzien und Hilfsreagenzien, in entsprechenden Schichten eines festen Trägers eingebettet oder fixiert. Diese Schichten werden als Nachweiselemente bezeichnet. Zur Bestimmung des entsprechenden Analyten wird die flüssige Probe mit diesen Nachweiselementen in Kontakt gebracht. Die Reaktion von flüssiger Probe und Reagenzien führt bei Anwesenheit eines Zielanalyten in der Regel zu einem optisch oder elektrochemisch nachweisbaren Signal, insbesondere einem Farbumschlag, welcher visuell oder mit Hilfe eines Geräts, meist reflexionsphotometrisch, ausgewertet werden kann. Andere Nachweismethoden beruhen beispielsweise auf elektrochemischen Methoden und erfassen Ladungs-, Potential- oder Stromänderungen.

Testelemente oder Testträger sind häufig als Teststreifen ausgebildet, die im wesentlichen aus einer länglichen Tragschicht aus Kunststoffmaterial und darauf angebrachten Nachweiselementen als Testfeldern bestehen. Es sind jedoch auch Testträger bekannt, die als quadratische oder rechteckige Plättchen gestaltet sind.

Testelemente für die klinische Diagnostik sind häufig so aufgebaut, dass der Probenaufgabebereich und der Detektionsbereich in einer vertikalen Achse stapelartig übereinander angeordnet liegen. Diese Konstruktionsweise birgt eine Reihe von Problemen. Wenn der probenbeladene Teststreifen zur Vermessung in ein Gerät, beispielsweise ein Reflexionsphotometer, eingebracht werden muss, kann potentiell infektiöses Probenmaterial mit Geräteteilen in Berührung kommen und diese gegebenenfalls kontaminieren. Daher ist eine räumliche Trennung von Probenaufgabebereich und Nachweiselement wünschenswert.

Vor allem in den Fällen, in denen die Teststreifen von ungeschulten Personen benutzt werden, beispielsweise bei der Blutzuckerselbstkontrolle oder Koagulationsselbstkontrolle, ist eine Volumendosierung in diesen Testelementen oft nur schwer zu realisieren. Der hierfür notwendige Transport der Flüssigkeitsprobe vom Probenaufgabebereich zum Nachweiselement stellt oft einen kritischen Prozess hinsichtlich der Dosierung der zu analysierenden Flüssigkeit und damit der Reproduzierbarkeit der Messung dar. Derartige Testelemente benötigen zusätzliche Vorrichtungen wie Kanäle, Membranen, Papiere oder Vliese zum Transport und zur Verteilung der Flüssigkeitsproben. Aufgrund dieses Aufbaus sind oftmals verhältnismäßig große Probenvolumina nötig, um zuverlässige Messungen zu ermöglichen. Für den Fall, dass beispielsweise Blut als Probenflüssigkeit eingesetzt wird, ist die Blutgewinnung für den Patienten umso schmerzhafter, je mehr Blut als Probenflüssigkeit gewonnen werden muss. Es wird deshalb generell angestrebt, Teststreifen zur Verfügung zu stellen, die mit möglichst wenig Probenmaterial auskommen. Weiterhin soll der Flüssigkeitstransport möglichst schnell erfolgen, um möglichst kurze Messzeiten zu erreichen.

Bei der Verwendung von Vliesen, Papieren oder Membranen zum Flüssigkeitstransport wird die Transportgeschwindigkeit durch die Eigenschaften des jeweiligen Materials entscheidend beeinflusst, so dass keine gleichmäßig hohen Transportgeschwindigkeiten garantiert werden können. Weiterhin haben die zuvor genannten Materialien den großen Nachteil, dass sie ein nicht unerhebliches Eigenvolumen besitzen und aufgrund ihrer mikroskopischen Struktur selbst kapillaraktiv sind.

So besitzen insbesondere Vliese und Papiere aufgrund ihrer Faserstruktur ein großes kapillaraktives Volumen, welches zwar die Verteilung der Flüssigkeit innerhalb des Materials und den Transport vom Probenaufgabebereich zum Nachweiselement aufgrund von Kapillarkräften ermöglicht, aber andererseits auch einen nicht unerheblichen Teil der zu untersuchenden Flüssigkeit zurückhält. Dadurch steht in derartigen Testelementen ein erheblicher Teil der ursprünglich aufgetragenen Probenflüssigkeit nicht für den eigentlichen Analytnachweis zur Verfügung, so dass größere Probenvolumina eingesetzt werden müssen, welche wiederum die oben genannten Nachteile für den Patienten mit sich bringen. Weiterhin tritt das Problem auf, dass es bei einer Anordnung von mehreren hintereinanderliegenden Nachweiselementen auf einem gemeinsamen Testelement zu einem nicht gleichförmigen und gleichzeitigen Reaktionsbeginn der in den Nachweiselementen stattfindenden Nachweisreaktionen kommt. Durch den relativ langsamen Flüssigkeitstransport durch kapillaraktive Vliese, Papiere oder Membranen erfolgt der Beginn der Nachweisreaktion in dem der Probenaufgabebereich zugewanden Nachweiselement deutlich eher als in Flussrichtung dahinterliegenden Nachweiselementen. Analoges gilt auch für das jeweilige Nachweiselement selbst. Hier erfolgt die Flüssigkeitsbenetzung und damit der Start der Nachweisreaktion zuerst an der dem Probenaufgabebereich zugewanden Seite, so dass es auch innerhalb eines Nachweiselementes zu Verzögerungen beim Start der Nachweisreaktion kommen kann. Somit kann es zu nicht gleichförmigen und nicht reproduzierbaren Reaktionsverläufen und damit fehlerhaften Analytbestimmungen kommen.

Bei mehreren hintereinanderliegenden Nachweiselementen kann es zusätzlich zu einer Verschleppung von Reagenzien aus einem Nachweiselement in benachbarte Nachweiselemente, welche in Flussrichtung dahinterliegen, und dadurch zu einer Verfälschung des Messergebnisses kommen.

Werden zum Transport der Flüssigkeitsproben vom Probenaufgabebereich zum Nachweiselement Kanalstrukturen eingesetzt, müssen diese bestimmte minimale und maximale Abmessungen bezüglich Breite, Höhe und Länge sowie Oberflächenbeschaffenheit des Kanals einhalten, um einen kapillaren Transport der Flüssigkeit zu ermöglichen. Hierdurch sind wiederum Beschränkungen bezüglich des zu transportierenden Flüssigkeitsvolumens beziehungsweise der Transportgeschwindigkeit gegeben.

Andere Mechanismen und Vorrichtungen zum Flüssigkeitstransport erfordern oft die Verwendung aktiver externer Kräfte wie beispielsweise Pumpen, so dass hierzu zusätzliche und damit kostenintensive Vorrichtungen benötigt werden.

Die bisher in Testelementen eingesetzten Kanäle haben oft den Nachteil, dass die ein nicht unerhebliches Innenvolumen besitzen, welches kapillaraktiv einen Teil des zu untersuchenden Flüssigkeitsvolumens zurückhält. Dadurch steht auch in derartigen Testelementen ein Teil der ursprünglich aufgetragenen Probenflüssigkeit nicht für den eigentlichen Analytnachweis zur Verfügung. Dies hat zur Folge, dass größere Probenvolumina eingesetzt werden müssen, welche wiederum die oben genannten Nachteile für den Patienten mit sich bringen.

Bisher in Testelementen eingesetzte Kanäle bestehen zumeist aus inerten Materialien, welche flüssigkeitsimpermeabel sind. In diesen Kanälen kann zwar ein rascher kapillarer Flüssigkeitstransport in den Bereich des Nachweiselements erfolgen, doch es sind weitere Strukturen und Vorrichtungen nötig, um die Flüssigkeit von der Kapillare in das Nachweiselement zu transportieren.

Eine Möglichkeit ist es, das Nachweiselement oder Teile dessen in direktem Kontakt zum Innenraum des kapillaren Kanals in diesen zu integrieren, so dass das Nachweiselement selbst einen Bestandteil des Kapillarkanals bildet. Dies hat allerdings den Nachteil, dass an den Stellen des Überganges von der Wand des Kapillarkanals zum Nachweiselement sprunghafte Übergänge in den Oberflächeneigenschaften der beiden Materialien auftreten können, welchen den Transport der Flüssigkeit behindern beziehungsweise komplett unterbrechen können. Hierdurch kann keine gleichmäßige und gleichzeitige Anströmung des Nachweiselements mit der zu untersuchenden Flüssigkeit garantiert werden. Stattdessen erfolgt die Benetzung und damit die Nachweisreaktion an dem dem Kapillarraum beziehungsweise dem Probenaufgabebereich zugewandten Bereichs des Nachweiselements eher als an davon abgewanden Stellen, wodurch der kontrollierte und gleichmäßige Ablauf der Nachweisreaktion und damit eine reproduzierbare Bestimmung des Analyten nicht erreicht werden kann.

Testelemente, welche den Übergang von kapillaren Kanal zum Nachweiselement mittels kapillaraktiver Materialien wie Vliesen oder ähnlichen Materialien, insbesondere sogenannten Spreitvliesen oder -geweben, herstellen, unterliegen ähnlichen Problemen und zusätzlich den zuvor genannten Nachteilen vliesähnlicher Materialien zum Flüssigkeitstransport.

EP-A-0 287 883 beschreibt ein Testelement, welches für die Volumendosierung einen kapillaren Zwischenraum zwischen Nachweisschicht und einem inerten Träger nutzt. Zur Befüllung des kapillaren Raumes wird das Testelement in die zu untersuchende Probe getaucht, was große Probenvolumina erforderlich macht, weshalb diese Form der Volumendosierung bevorzugt für die Untersuchung von im Überschuss vorhandenem Probenmaterial, beispielsweise Urin, geeignet ist. Der Kapillarraum dient hier ausschließlich zur Volumendosierung. Eine räumliche Trennung von Probenaufgabebereich und Detektionsort sowie ein durch den Kapillarspalt hervorgerufener gerichteter Flüssigkeitstransport zum Detektionsort hin ist in der beschriebenen Vorrichtung nicht vorgesehen. Weiterhin bildet in der beschriebenen Vorrichtung das Nachweiselement selbst einen Teil des Kapillarraums aus.

DE 197 53 847 beschreibt ein Testelement zur Bestimmung eines Analyten in einer Flüssigkeit, welches auf einem inerten Träger einen zum kapillaren Flüssigkeitstransport befähigten Kanal und ein Nachweiselement besitzt. Der zum kapillaren Flüssigkeitstransport befähigte Kanal ist erfindungsgemäß dadurch gekennzeichnet, dass er zumindest teilweise vom Träger und dem Nachweiselement gebildet wird und in Richtung des kapillaren Transports von der Probenaufgabeöffnung zumindest bis zu der der Entlüftungsöffnung nächstgelegenen Kante des Nachweiselements reicht. Nachteilig an dieser Ausführungsform ist insbesondere, dass das Nachweiselement ein direkter Bestandteil des zum kapillaren Flüssigkeitstransport befähigten Kanals ist. Hierdurch kann es durch die unterschiedlichen Oberflächeneigenschaften der einzelnen Komponenten des Kanals zu den oben erwähnten Problemen wie Beeinflussung beziehungsweise Abreißen des kapillaren Transports oder ungleichmäßiger Benetzung des Nachweiselements kommen. Weiterhin kommt die zu untersuchende Flüssigkeit im Kanal selbst mit den Reagenzien des Nachweiselements direkt in Kontakt, so dass in dieser Ausführungsform eine räumliche Trennung von Transportraum und Nachweisbereich nicht vorhanden ist.

Aus DE-A 31 51 291 ist ein Gerät zur Analyse von biologischen Fluiden bekannt, welches einen Träger mit einem selbstfüllbaren Messkanal sowie eine Laminatanordnung mit einer Filterschicht und einer Reagenzmaterialschicht aufweist. Die Probenflüssigkeit wird bei diesem Testträger durch Kapillarkräfte in den Testkanal transportiert und dringt von diesem in das darüberliegende Laminat ein, wo nach Erwärmen des analytischen Geräts eine Nachweisreaktion des Zielanalyten stattfindet. Als Filterschicht bildet in diesem Gerät eine mikroporöse Membran mit Porengrößen von weniger als 1 µm die obere Abdeckung des Kapillarkanals. Diese Filtermembran hat erfindungsgemäß die Funktion, die Reagenzmaterialschicht von störenden Bausteinen, beispielsweise Zellstrukturen, zu isolieren. Diese Filtermembran erfüllt vor allem die Aufgabe, die Flüssigkeitsprobe vor ihrer Analyse in der Reagenzmaterialschicht aufzuarbeiten und in ihrer Zusammensetzung zu verändern und ist somit schon am Nachweis des Analyten beteiligt. Nachteilig hierbei ist, dass durch die sehr geringen Porenöffnungen von weniger als 1 µm nur ein sehr langsames Eindringen der Flüssigkeit in die Reagenzmaterialschicht erfolgen kann, was lange Messzeiten mit sich bringt. Insbesondere bei der Verwendung von Lösungen, welche, wie beispielsweise Blut, Partikel oder Zellen in hohen Konzentrationen enthalten, kann es durch die geringe Porengröße der Membran leicht zu einem Verstopfen der Filtermembran und somit zu einer Beeinträchtigung oder Unterbrechung des Transports der zu untersuchenden Flüssigkeit in den Detektionsbereich kommen. Hierdurch ist die Durchführbarkeit und Reproduzierbarkeit der Analytbestimmung nicht in allen Fällen gewährleistet. Weiterhin ist nachteilig, dass zur Bestimmung des Analyten das analytische Gerät mit der darin enthaltenen Probe erhitzt werden muss. Dadurch ist die Benutzung des analytischen Geräts im wesentlichen auf Labors beschränkt.

DE 196 29 657 beschreibt einen diagnostischen Testträger, welcher auf einer Tragschicht eine oder mehrere Nachweisschichten und ein die Nachweisschichten überdeckendes Netzwerk, das größer als die Nachweisschichten und auf der Tragschicht befestigt ist, enthält. Zur Bestimmung von Analyten wird die zu untersuchende Flüssigkeit direkt auf das Netz aufgebracht und fließt durch dieses in die Nachweisschichten hindurch. Probenaufgabebereich und Nachweisschichten sind hier in einer vertikalen Achse übereinander angeordnet, was die zuvor genannten Probleme solcher stapelartiger Anordnungen hervorruft. Ein gezielter Transport der zu untersuchenden Flüssigkeit vom Probenaufgabebereich zu einer davon in horizontaler Richtung entfernt liegenden Nachweisschicht, beispielsweise mittels kapillarer Kanäle, findet vor der Bestimmung des Analyten nicht statt. Das Netzwerk hat hier die Aufgabe, überschüssige Flüssigkeit durch das Netzwerk von der Nachweisschicht in den über die Nachweisschicht hinausragenden Teil des Netzwerks wegzuführen. Die Dicke des Netzwerkes soll vorzugsweise hierzu so beschaffen sein, dass sich die darauf liegende Abdeckung und die darunter liegende Tragschicht sich in einem solchen Abstand voneinander befinden, dass verbleibende Flüssigkeit über der gesättigten Nachweisschicht und in den gefüllten Maschen des Netzwerkes durch Kapillarkraft in den Bereich unter der Abdeckung aufgesaugt und vom Probenaufgabebereich weggeführt wird. Der Flüssigkeitstransport erfolgt in diesen Bereichen lateral durch Kapillarkräfte innerhalb des Netzwerkes selbst beziehungsweise durch Kapillarkräfte zwischen Netzwerk und Abdeckung beziehungsweise Tragschicht, jedoch nicht durch Kapillarkräfte, in denen das Netzwerk eine Wandung eines größeren Kapillarspaltes bildet. Da das Netzwerk in der beschriebenen Vorrichtung andere Anforderungen erfüllen muss, besitzt es im Vergleich zu dem Netzwerk der vorliegenden Erfindung weiterhin unterschiedliche geometrische Parameter und Materialeigenachaften.

US 4,323,536 beschreibt ein analytisches Testelement, bei welchem über einen Kapillarkanal verschiedene Nachweiselemente mit Probenflüssigkeit angeströmt werden können. Diese Nachweiselemente bestehen aus kapillaraktiven Materialien, welche die Probenflüssigkeit aufsaugen und in welchen die Nachweis- und Detektionsprotesse ablaufen. Diese kapillaraktiven Nachweiselemente stehen entweder in direktem Kontakt mit dem Probenkanal oder es können weitere Schlichten, beispielsweise Filterschichten, zwischengeschaltet sein.

US 6,592,815 bzw. DE 197 538 49 A1 beschreibt ein analytisches Testelement, welches ein kapillaraktives Vliesmaterial enthält, das die Probenflüssigkeit aus einem Kapillarspalt des Testelements aufsaugen kann. Die Probenflüssigkeit wird innerhalb des Vliesmaterials in alle Raumrichtungen weitergeleitet und so in diesem verteilt. Nachfolgend gibt das mit Probenflüssigkeit getränkte Vliesmaterial eine Teilmenge der Probenflüssigkeit an die angrenzenden Nachweiselemente weiter, in welchen dann die eigentlichen Nachweis- und Detektionsprozesse ablaufen.

### Aufgabe der Erfindung

Die Aufgabe der vorliegenden Erfindung ist es, die Nachteile des Standes der Technik zu beseitigen. Insbesondere soll ein einfach zu handhabendes, selbständig volumendosierendes Testelement zu Verfügung gestellt werden, mit dem unter Verwendung minimaler Probenvolumina eine räumliche Trennung von Detektionsbereich und Probenaufgabebereich möglich ist. Zusätzlich soll der Flüssigkeitstransport vom Probenaufgabebereich zum Detektionsbereich möglichst schnell und vollständig erfolgen, so dass die Analyse einer Probe zeitlich dadurch nicht limitiert wird. Insbesondere soll bei Testelementen, welche mehrere Nachweiselemente besitzen, gewährleistet sein, dass die Flüssigkeitsprobe möglichst zeitgleich und ohne Verschleppungsprobleme die einzelnen Nachweiselemente erreicht und damit die einzelnen Nachweisreaktionen möglichst gleichzeitig beginnen können. Des weiteren soll durch einen einfachen Aufbau des Testelements eine kostengünstige und produktionstechnisch einfach zu realisierende Fertigung ermöglicht werden.

Dies wird durch den Gegenstand der Erfindung, wie er in den Patentansprüchen und der Beschreibung charakterisiert ist, erreicht.

### Erfindungsgemäße Lösung

Gegenstand der Erfindung ist ein analytisches Testelement zur Bestimmung mindestens eines Analyten in einer Flüssigkeit, wie in Anspruch 1 definiert. Weitere Gegenstände der Erfindung sind die Verwendung eines solchen analytischen Testelements zur Bestimmung eines Analyten in einer Flüssigkeit sowie Verfahren zur Bestimmung eines Analyten in einer Flüssigkeit mit Hilfe eines solchen analytischen Testelements.

Der zum kapillaren Flüssigkeitstransport befähigte Kanal wird erfindungsgemäß zumindest teilweise von einem hydrophilen Netzwerk begrenzt wie in Anspruch 1 definiert. Erfindungsgemäß befinden sich ein oder mehrere Nachweiselemente in direktem Kontakt zu der dem Kapillarspalt abgewanden Seite des Netzwerkes. Das Netzwerk liegt folglich, zumindest in Teilbereichen, zwischen dem Kapillarspalt und dem Nachweiselement. Das Netzwerk stellt eine Begrenzungsfläche des Kapillarspaltes dar und ermöglicht den Transport der Flüssigkeitsprobe vom Probenaufgabebereich durch den Kapillarspalt hindurch zu den Bereichen des Kapillarspaltes, welche sich, durch das Netzwerk getrennt, unterhalb des Nachweiselementes befinden. Die Oberflächeneigenschaften und geometrischen Größen des Netzwerkes und des kapillaren Kanals bestimmen hierbei entscheidend die erfindungsgemäße Funktionsweise des Testelements mit. So erfolgt beispielsweise in einer bevorzugten Ausführungsform der Transport der Flüssigkeitsprobe vom Probenaufgabebereich durch den kapillaren Spalt bis zur gegenüberliegenden Entlüftungsöffnung innerhalb weniger Sekunden, ohne dass zunächst Flüssigkeit über das Netzwerk hinaus den Kapillarspalt verlässt. In dieser Ausführungsform kann beispielsweise ein Bluttropfen von 30 µl Volumen innerhalb von ca. 3 - 5 Sekunden den gesamten Kapillarspalt, welcher eine Breite von ca. 2 mm, eine Höhe von ca. 200 µm und eine Länge von ca. 25 mm besitzt, füllen. Hierdurch ist eine sehr rasche und weitgehend zeitgleiche Anströmung der Nachweiselemente mit der Flüssigkeitsprobe möglich.

Die Flüssigkeit kann erfindungsgemäß unter normalen Bedingungen wie Atmosphärendruck nur an den Stellen, an welchen auf der anderen Seite des Netzwerkes ein oder mehrere Nachweiselemente angeordnet sind, durch das Netzwerk hindurchtreten und in die jeweiligen Nachweiselemente einfließen. Hierzu ist ein direkter Kontakt der Nachweiselemente mit der dem Kapillarspalt abgewanden Seite des Netzwerkes nötig.

Überraschenderweise zeigte sich, dass bei geeigneten Oberflächeneigenschaften und geometrischen Größen des Netzwerkes, des Kapillarspaltes und der Nachweiselemente und einer geeigneten Anordnung der einzelnen Elemente zueinander sowohl eine sehr schnelle Befüllung des Kapillarspalts als auch anschließend eine weitgehend gleichmäßige und gleichzeitige Benetzung der Nachweiselemente erfolgt. Die Präzision und Reproduzierbarkeit der Analytbestimmung wird damit erhöht.

Die geometrischen Abmessungen und das Volumen des zum kapillaren Flüssigkeitstransports befähigten Kanals oder Kapillarspalts können an die zu untersuchenden Probenvolumina angepasst werden. Für den bevorzugten Fall, dass der Kanal einen im wesentlichen rechteckigen Querschnitt aufweist, ist eine Dimension, beispielsweise die Höhe des Kanals, durch die physikalischen Grenzen der Kapillaraktivität vorgegeben. Das Volumen des kapillaren Kanals lässt sich dann durch geeignete Wahl der beiden übrigen Dimensionen, beispielsweise Länge und Breite, einstellen. Die Höhe der Kapillare liegt beispielsweise für wässrige Flüssigkeiten in der Größenordnung von 10 bis 500 µm, bevorzugt zwischen 20 und 300 µm, ganz bevorzugt zwischen 50 und 200 µm. Je nach gewünschtem Volumen kann die Breite dann mehrere mm, bevorzugt 1 bis 5 mm, ganz bevorzugt 1 bis 3 mm, und die Länge bis zu einigen cm, bevorzugt 0,5 bis 5 cm, ganz bevorzugt 1 bis 3 cm betragen. Allerdings sind die kapillaren Eigenschaften eines Kanals nicht nur von den geometrischen Abmessungen abhängig, sondern auch von anderen Parametern wie Oberflächenbeschaffenheit und Hydrophobizität der Kanalwände oder den rheologischen Eigenschaften der Probenflüssigkeit. Die hier genannten Abmessungen sind somit nicht als beschränkend, sondern vielmehr als beispielhaft zu sehen. Die optimalen Abmessungen und Oberflächeneigenschaften des Kapillarspaltes können von einem Fachmann so bestimmt werden, dass die Beschaffenheit des Kanals an die jeweiligen Erfordernisse angepasst werden kann.

Der Kapillarspalt besteht teilweise aus einer Trägersubstanz, die so ausgeformt ist, dass sie insbesondere an den Stellen, an welchen sie mit dem Netzwerk bedeckt ist, einen Kapillarkanal ausbilden kann. In einer bevorzugten Ausführungsform ist hierzu eine entsprechende Vertiefung in die Trägersubstanz eingebracht, z. B. geprägt, geätzt oder gefräst. In einer weiteren Ausführungsform wird die Geometrie des Kapillarkanals nicht primär von der Form der Trägersubstanz, sondern maßgeblich von zusätzlichen Zwischenschichten bestimmt. Bevorzugt werden hierzu ein oder mehrere Abstandshalter derartig auf einem Träger angebracht, dass sich die den späteren kapillaren Kanal zugewanden Seiten in einem Abstand voneinander befinden, welcher der Breite des späteren Kapillarspaltes entspricht. Die Höhe des späteren Kapillarspaltes ist hierbei durch die Höhe der Abstandshalter vorgegeben. Die Länge des späteren Kapillarspaltes kann durch die Länge der Abstandshalter vorgegeben sein.

Die Zwischenschicht kann besonders vorteilhaft aus einem doppelseitig klebenden Band gefertigt sein, das neben der Bestimmung der Kapillarkanalgeometrie ebenfalls das Verbinden der übrigen an der Bildung der kapillaraktiven Zone beteiligten Komponenten, also Träger und Netzwerk, ermöglicht.

In einer bevorzugten Ausführungsform wird die Form des späteren Kapillarkanals durch die Ausgestaltung der Zwischenschicht beziehungsweise der Abstandshalter vorgegeben. So können beispielsweise durch Stanzen oder Schneiden des Abstandshaltermaterials bestimmte Bereiche derartig ausgestaltet sein, dass die als spezielle Probenaufgabebereiche oder Entlüftungsöffnungen dienen.

In einer weiteren Ausführungsform werden als Zwischenschicht und Abstandshalter zwei doppelseitig klebende Bänder auf einem Träger in einem Abstand zueinander angebracht, welcher der Breite des Kapillarkanals entspricht. Durch Aufbringen des Netzwerkes auf die doppelseitig klebenden Bänder kann so ein Kapillarkanal gebildet werden.

Für die Wandungen des Kapillarspaltes, insbesondere für das Trägermaterial und eventuelle Zwischenschichten, werden vorzugsweise inerte Materialien, welche die zu untersuchenden Flüssigkeiten nicht aufnehmen, eingesetzt. Dies sind insbesondere nicht saugfähige Materialien, wobei Kunststoffolien beispielsweise aus Polystyrol, Polyvinylchlorid, Polyester, Polycarbonat oder Polyamid besonders bevorzugt sind. Als weitere Trägermaterialien eignen sich Metallfolien, Keramik oder Glas. Es ist jedoch auch möglich, saugfähige Materialien, wie zum Beispiel Holz, Papier oder Pappe mit wasserabstoßenden Mitteln zu imprägnieren.

Der Kapillarspalt wird an seinen Enden von einer Probenaufgabeöffnung und einer Entlüftungsöffnung begrenzt. Die Probenaufgabeöffnung ist vorzugsweise derartig ausgeformt, dass sie sicherstellt, dass die Probenflüssigkeit in den Kapillarkanal eintritt. Dies kann beispielsweise dadurch erreicht werden, dass der Probentropfen direkt mit einer der Flächen in Kontakt gebracht werden kann, welche eine der inneren Oberflächen der Kapillare bildet, insbesondere durch Inkontaktbringen des Flüssigkeitstropfens mit dem entsprechenden Ende des Kapillarspalts. Die Probenaufgabeöffnung kann aber auch durch eine Aussparung in mindestens einer der den Kapillarspalt bildenden Wände gebildet sein. Durch geeignete Wahl der Geometrie und Dimensionen der Aussparung wird erreicht, dass der Flüssigkeitstropfen unabhängig von der genauen Position des Auftragens mit sehr hoher Wahrscheinlichkeit mit der kapillaraktiven Zone in Kontakt kommt und bereitwillig in das Innere der Kapillare eingesaugt wird. Beispielsweise ist die Größe der frei liegenden Fläche so zu wählen, dass ein auf sie aufgebrachter Flüssigkeitstropfen zumindest an einer Stelle mit der kapillaraktiven Zone in Kontakt kommt. Beispielsweise ist eine Dimension der Aussparung, z. B. deren Breite, so zu wählen, dass der Durchmesser des Flüssigkeitstropfens geringfügig größer ist als die gewählte Dimension der Aussparung. Für einen Tropfen von 3 µl hat sich beispielsweise eine Breite der Aussparung von 1 mm als geeignet herausgestellt, für größere Flüssigkeitsmengen entsprechend größere Aussparungen. Besonders bevorzugt wird das Einsaugen des Probentropfens in den kapillaren Kanal dadurch erreicht, dass die durch die Aussparung frei liegende Fläche hydrophiliert ist und zumindest in Richtung des kapillaren Transportkanals direkt in die kapillaraktive Zone übergeht. Eine bevorzugte Möglichkeit, eine solche speziell ausgeformte Probenaufgabeöffnung oder Entlüftungsöffnung zu erzeugen, ist die Verwendung speziell gestanzter oder zugeschnittener Zwischenschichten, beispielsweise doppelseitig klebender Bänder, in zuvor beschriebener Weise. Eine solche Ausgestaltungsform ist beispielsweise in WO 03/095092. Weitere bevorzugte Probenaufgabeöffnungen können so geformt sein, dass die direkt in Kontakt mit einem Tropfen der Probenflüssigkeit gebracht werden können und diesen Tropfen dann durch Kapillarkräfte zu den Nachweiselementen transportieren. Eine derartige Ausgestaltungsform ist beispielsweise in DE 197 53 850 A1 beschrieben.

Die Bereitschaft einer Kapillare, eine Flüssigkeit aufzusaugen, geht mit der Benetzbarkeit der Kanaloberfläche mit der Flüssigkeit einher. Für wässrige Proben bedeutet dies, dass eine Kapillare aus einem Material gefertigt werden sollte, dessen Oberflächenspannung nahe an die Oberflächenspannung von Wasser, welche 72 mN/m beträgt, heranreicht oder diesen Wert übertrifft. Hydrophile Oberflächen sind in diesem Zusammenhang wasseranziehende Flächen. Wässrige Proben, darunter auch Blut, spreiten auf solchen Oberflächen gut. Solche Flächen sind unter anderem dadurch charakterisiert, dass an der Grenzfläche ein Wassertropfen auf ihnen einen spitzen Rand- oder Kontaktwinkel ausbildet. Im Gegensatz dazu wird auf hydrophoben, das heißt wasserabweisenden Oberflächen, an der Grenzfläche zwischen Wassertropfen und Oberfläche ein stumpfer Randwinkel ausgebildet.

Ausreichend hydrophile Materialien zum Aufbau einer Kapillare, die schnell wässrige Proben aufsaugt, sind beispielsweise Glas, Metall oder Keramik. Für den Einsatz in Testträgern sind diese Materialien jedoch nur bedingt geeignet, da sie einige Nachteile aufweisen, beispielsweise Bruchgefahr bei Glas oder Keramik, oder Veränderung der Oberflächeneigenschaften mit der Zeit bei zahlreichen Metallen. Üblicherweise werden deshalb zur Fertigung von Testelementen Kunststofffolien oder -formteile eingesetzt.

Die verwendeten Kunststoffe übertreffen dabei in der Regel kaum eine Oberflächenspannung von 45 mN/m. Selbst mit den hydrophilsten gängigen Kunststoffen wie beispielsweise Polymethylmethacrylat oder Polyamid lassen sich, wenn überhaupt, nur sehr langsam saugende Kapillaren aufbauen. Kapillaren aus hydrophoben Kunststoffen wie beispielsweise Polystyrol, Polypropylen oder Polyethylen saugen im wesentlichen keine wässrigen Proben. Hieraus ergibt sich die Notwendigkeit, Kunststoffe für die Verwendung als Konstruktionsmaterial für Testelemente mit kapillaraktiven Kanälen hydrophil auszustatten, das heißt zu hydrophilieren.

In einer bevorzugten Ausführungsform des erfindungsgemäßen analytischen Testelements ist zumindest eine, besser jedoch zwei, ganz besonders bevorzugt zwei sich gegenüberliegende Flächen der die innere Oberfläche des zum kapillaren Flüssigkeitstransports befähigten Kanals bildenden Flächen, hydrophiliert. Insbesondere bildet das hydrophile Netzwerk solch eine hydrophile innere Oberfläche des Kapillarspaltes.

Wird mehr als eine Fläche hydrophiliert, so können die Flächen entweder mit der gleichen oder mit unterschiedlichen Methoden hydrophil gemacht werden.

Die Hydrophilisierung ist vor allem dann notwendig, wenn die Materialien, die den kapillaraktiven Kanal bilden, insbesondere der Träger, selbst hydrophob oder nur sehr wenig hydrophil sind, beispielsweise weil sie aus unpolaren Kunststoffen bestehen. Unpolare Kunststoffe, wie zum Beispiel Polystyrol, Polyethylen, Polyethylenterephthalat oder Polyvinylchlorid, sind als Trägermaterialien vorteilhaft, weil sie die zu untersuchenden Flüssigkeiten nicht absorbieren und damit das Probenvolumen effektiv zur Analytbestimmung genutzt werden kann. Durch die Hydrophilisierung der Oberfläche des Kapillarkanals wird erreicht, dass eine wässrige Probenflüssigkeit bereitwillig in den kapillaren Kanal eintritt und dort rasch zum Nachweiselement transportiert wird.

Idealerweise wird die Hydrophilisierung der Oberfläche des kapillaren Kanals dadurch erreicht, dass zu seiner Fertigung ein hydrophiles Material eingesetzt wird, das jedoch die Probenflüssigkeit selbst nicht oder nicht wesentlich aufzusaugen vermag. Wo dies nicht möglich ist, kann die Hydrophilisierung einer hydrophoben oder nur sehr wenig hydrophilen Oberfläche durch geeignete Beschichtung mit einer stabilen, gegenüber dem Probenmaterial inerten, hydrophilen Schicht erreicht werden, beispielsweise durch kovalente Bindung von photoreaktiv ausgerüsteten, hydrophilen Polymeren auf eine Kunststoffoberfläche, durch Aufbringen netzmittelhaltiger Schichten oder durch Beschichtung von Oberflächen mit Nanokompositen mittels Sol-Gel-Technologie. Auch ein Aufbringen ganzer hydrophiler Oberflächen, beispielsweise in Form von Follen, auf hydrophobe Träger kann diese hydrophilisieren. Darüber hinaus ist es möglich, durch thermische, physikalische oder chemische Behandlung der Oberfläche eine gesteigerte Hydrophilie zu erzielen, beispielsweise durch Behandlung der Oberfläche mit Netzmitteln wie Dioctylnatriumsulfosuccinat oder Oleoylsarkosinsäure.

Für as Netzwerk des erfindungsgemäßen diagnostischen Testträgers werden monofile Gewebe eingesetzt, die hydrophil sind. Hierfür kann das Gewebematerial selbst hydrophil sein oder es kann, beispielsweise durch Behandlung mit Netzmittel, hydrophil gemacht werden.

Die Maschenweite des Netzwerkes spielt eine entscheidende Rolle für eine optimale Funktion des erfindungsgemäßen Testelements, wie in Anspruch 1 definiert,

Erfindunpgemäß wurde gefunden, dass Netzwerke mit einer Maschenweite zwischen 10 und 500 µm, bevorzugt zwischen 20 und 300 µm, besonders bevorzugt zwischen 50 und 150 µm sowie einer Dicke zwischen 10 und 500 µm, bevorzugt zwischen 20 und 300 µm, besonders bevorzugt zwischen 50 und 150 µm hierfür besonders geeignet sind. Das Netzwerk besteht aus einem hydrophilem Gewebe. Überraschenderweise hat es sich herausgestellt, dass auch nicht hydrophiles, aber dafür gut bearbeitbares und preiswertes Gewebe eingesetzt werden kann, wenn eine Hydrophilisierung der Oberfläche des Gewebes erfolgt. Als besonders bevorzugtes Netzmaterial wird Polyethylenterephthalat verwendet, wobei das Netzwerk aus diesem Material anschließend mit einem Netzmittel wie z. B. Dioctylnatriumsulfosuccinat oder Oleoylsarkosinsäure behandelt und dadurch hydrophilisiert wird.

Bevorzugt weist das Netzwerk einen Faser- oder Drahtdurchmesser zwischen 10 und 300 µm, bevorzugt zwischen 30 und 150 µm, besonders bevorzugt zwischen 50 und 100 µm auf.

Der Begriff Netzwerk ist im Sinne der vorliegende Erfindung auf monofile Gewebe beschränkt.

Das Netzwerk muss nicht notwendigerweise eine gesamte Seitenfläche des Kapillarspalts ausbilden, um die erfindungsgemäße Funktion des Testelementes zu ermöglichen. In bevorzugten Ausführungsformen kann das Netzwerk nur einen Teil der Wandungen des Kapillarspaltes ausbilden. In diesen Fällen muss das Netzwerk jedoch mindestens teilweise mit dem Innenraum des Kapillarspalts und mindestens teilweise mit dem Nachweiselement in direktem Kontakt stehen, so dass ein Flüssigkeitstransport von Innenraum des Kapillarspaltes in das Nachweiselement möglich ist. Besonders bevorzugt sind derartige Ausführungsformen, wenn das Netzwerk selbst teuer ist. Hier können solche Ausführungsformen, in welchen das Netzwerk lediglich in den Bereichen der Nachweiselemente am Aufbau des Kapillarspaltes beteiligt ist, zu einer deutlichen Reduktion der Herstellungskosten führen.

Der erfindungsgemäße Einsatz eines kapillaraktiven Kanals mit einem Netzwerk als Begrenzung zeigt überraschenderweise folgende Vorteile:
- Da der zum kapillaren Flüssigkeitstransport befähigte Kanal sich in sehr kurzer Zeit mit der zu untersuchenden Flüssigkeit füllen und diese über das Netzwerk weitgehend gleichmäßig und gleichzeitig in die angrenzenden Nachweiselemente gelangen kann, ist gewährleistet, dass eine inhomogene Benetzung des Nachweiselements mit der Flüssigkeitsprobe und damit eine Verfälschung der Messung vermieden wird.
- Es kann eine weitgehend gleichmäßig und gleichzeitig ablaufende Nachweisreaktion erzielt werden.
- Weiterhin kann durch die geometrischen Abmessungen des Kapillarkanals das darin enthaltene Flüssigkeitsvolumen genau und reproduzierbar bestimmt werden. Der erfindungsgemäße Kapillarspalt erfüllt somit auch Aufgaben der Volumendosierung der Probenflüssigkeit. Die Präzision und Reproduzierbarkeit der Messung wird somit erhöht.

Bei Testelementen, bei welchen in einem räumlich genau definierten Bereich der Verlauf oder das Ergebnis einer Nachweisreaktion im Nachweiselement detektiert wird, beispielsweise bei optischer Detektion in einem speziellen Gerät, und bei welchen daher eine Trennung von Probenaufgabebereich und Detektionszone, beispielsweise aus Gründen der Gerätehygiene, angestrebt ist, hat die Verwendung eines kapillaraktiven Kanals zum Flüssigkeitstransport weiterhin den Vorteil, dass ein sehr rascher Transport der Flüssigkeitsprobe von der Probenaufgabeöffnung im Testelement zur Detektionsstelle im Nachweiselement erfolgt. Somit wird hierdurch die Analyse der Probe zeitlich nicht limitiert. Außerdem wird durch eine solche Anordnung eine bequemere Anwendung für den Benutzer erreicht.

Neben den bereits genannten Vorteilen weist das erfindungsgemäße Testelement weitere Vorzüge auf. Durch die räumliche Trennung von Probenaufgabebereich und Signaldetektion in Verbindung mit der Probenvolumendosierung wird eine hygienische Handhabung des Probenmaterials erreicht. Vor allem bei optischer Detektion, beispielsweise mittels eines Reflexionsphotometers, wird eine Kontamination des Gerätes weitestgehend ausgeschlossen, da die Probe beispielsweise auf ein aus dem Gerät herausragendes Testelement aufgegeben werden kann, die zur Bestimmung des Analyten erforderliche Menge an Probe in den kapillaren Kanal eingesaugt wird und selbständig und dosiert ohne weitere Maßnahmen zu der im Geräteinneren gelegenen Detektionszone des Testelements transportiert wird.

Ein Nachweiselement enthält für die Nachweisreaktion des Zielanalyten in der Probe notwendigen Reagenzien sowie gegebenenfalls Hilfsstoffe. Das Nachweiselement kann auch nur Teile der Reagenzien oder Hilfsstoffe enthalten. Dem mit der Technik von analytischen Testelementen oder diagnostischen Testträgern vertrauten Fachmann sind solche Reagenzien und Hilfsmittel bestens bekannt. Für Analyten, die enzymatisch nachzuweisen sind, können beispielsweise Enzyme, Enzymsubstrate, Indikatoren, Puffersalze, inerte Füllstoffe und dergleichen mehr im Nachweiselement enthalten sein. Das Nachweiselement kann aus einer oder mehreren Schichten aufgebaut sein und weiterhin eine Abdeckung, bevorzugt auf der Seite des Nachweiselements, die nicht mit der Probe in Kontakt gebracht wird, enthalten. Für den besonders bevorzugten Fall, dass die Nachweisreaktion zu einer beobachtbaren Farbveränderung führt, worunter in diesem Zusammenhang entweder die Änderung einer Farbe, das Entstehen einer Farbe oder das Verschwinden von Farbe verstanden werden soll, ist sicherzustellen, dass der Träger durch geeignete Maßnahmen eine visuelle oder optische Beobachtung der Nachweisreaktion zulässt. Dazu kann das Trägermaterial und das Netzwerk oder eine eventuelle Abdeckung des Nachweiselements selbst durchsichtig sein oder das Trägermaterial und das Netzwerk oder eine eventuelle Abdeckung des Nachweiselements kann auf der Detektionsseite eine durchsichtige Aussparung besitzen. Ist das Nachweiselement nicht von einer Abdeckung umgeben, kann die Farbveränderung des Nachweiselementes auch direkt bestimmt werden, vorzugsweise mittels reflektionsphotometrischer Bestimmung.

Neben Nachweisreaktionen, die zu Farbveränderungen führen, sind dem Fachmann auch andere Nachweisprinzipien bekannt, die mit dem beschriebenen Testelement realisiert werden können, beispielsweise elektrochemische Sensoren oder chemische, biochemische, molekularbiologische, immunologische, physikalische, fluorimetrische oder spektroskopische Nachweismethoden.

Für das Nachweiselement ist es erforderlich, solche Materialien einzusetzen, die in der Lage sind, die zu untersuchende Flüssigkeit mit den enthaltenen Analyten aufzunehmen. Dies sind insbesondere saugfähige Materialien wie beispielsweise Vliese, Gewebe, Gewirke, Papiere oder poröse Kunststoffmaterialien. Die dafür in Frage kommenden Materialien müssen Reagenzien tragen können, die den Nachweis des zu bestimmenden Analyten ermöglichen. Bevorzugte Materialien für das Nachweiselement sind Papiere oder poröse Kunststoffmaterialien, wie Membranen. Als poröse Membranmaterialien sind Polyamid-, Polyvinylidendifluorid-, Polyethersulfon- oder Polysulfonmembranen ganz besonders bevorzugt. Die Reagenzien zur Bestimmung des nachzuweisenden Analyten können vorzugsweise durch Imprägnierung in die vorstehend genannten Materialien eingebracht werden.

Für das Nachweiselement kommen besonders bevorzugt sogenannte offene Filme in Frage, wie sie beispielsweise in EP-B-0 016 387 beschrieben sind. Hierfür werden einer wässrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wässrigen Dispersionen. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Mögliche Reagenzien, welche zur Bestimmung eines bestimmten Analyten eingesetzt werden können, sind dem Fachmann bekannt.

Das Nachweiselement kann weiterhin über Bestandteile verfügen, die einen Ausschluss störender Probenanteile von der Nachweisreaktion erlauben und somit als Filter, beispielsweise für partikuläre Probenbestandteile wie Blutzellen, wirken. Für visuelle oder optische Detektionsverfahren ist beispielsweise bei der Analyse von Blutproben der rote Blutfarbstoff Hämoglobin, der in den Erythrozyten enthalten ist, störend. Zweckmäßigerweise werden diese störenden Komponenten vor der eigentlichen Detektionsreaktion von der Probe, beispielsweise Vollblut, abgetrennt. Dies kann durch Probenaufbereitung vor dem Auftragen der Probe auf das Testelement geschehen, wie zum Beispiel durch Zentrifugation von Vollblut und anschließender Serum- oder Plasmagewinnung. Bequemer und auch für den Anwender einfacher ist es, wenn das Testelement diesen Trennungsschritt durch geeignete Konstruktion selbst durchführt. Dem Fachmann sind Mittel aus der Teststreifentechnologie bekannt, die einen zuverlässigen Ausschluss von Erythrozyten und anderen störenden Blutbestandteilen gewährleisten. Beispielsweise ist die Verwendung von semipermeablen Membranen oder Glasfaservliesen, wie sie beispielsweise aus EP-B-0 045 476 bekannt sind, zur Abtrennung roter Blutkörperchen möglich.

Der Kontakt des Nachweiselementes zum Netzwerk kann auf dem Fachmann bekannte Weise hergestellt werden. Als besonders bevorzugt hat es sich erwiesen, die Nachweiselemente zunächst auf das Netzwerk aufzugeben und dann zumindest zwei seitliche, vorzugsweise gegenüberliegende, Randbereiche des Nachweiselements mit dem Netzwerk zu verkleben. Besonders bevorzugt ist die Befestigung mindestens an den Seiten, welche senkrecht zum Verlauf des Kapillarspaltes stehen. Hierdurch kann eine Fixierung und ein direkter Kontakt des Nachweiselementes mit dem Netzwerk erreicht werden, ohne dass die Kontaktfläche selbst durch Klebstoffe verunreinigt wird. In ein besonders bevorzugten Ausführungsform wird dieses Verkleben durch Schmelzkleber erzielt. Weitere Kontaktierungs- und Befestigungsverfahren, wie beispielsweise Verschweißen, sind dem Fachmann bekannt.

Die Abmessungen des Nachweiselements müssen nicht unbedingt mit den Abmessungen des Kapillarkanals bzw. des Netzwerkes übereinstimmen. Insbesondere kann das Nachweiselement Bereiche enthalten, welche zwar mit dem Netzwerk verbunden sind, aber nicht mit dem Kapillarspalt in fluidischer Verbindung stehen. Solche Bereiche sind beispielsweise Bereiche des Nachweiselementes, welche sich über Seitenbegrenzungen des Kapillarspaltes befinden. In solchen Fällen hat es sich als zweckmäßig erwiesen, Nachweiselemente einzusetzen, welche selbst für eine weitere Verteilung der Flüssigkeit über das gesamte Volumen des Nachweiselements sorgen. Solche Nachweiselemente können beispielsweise Vliesstrukturen besitzen, welche eine weitere Verteilung der Flüssigkeitsprobe innerhalb des Nachweiselements bewirken. Der Flüssigkeitstransport durch das Netzwerk dient in diesem Fällen vor allem zum Herstellen eines Flüssigkeitskontaktes zwischen Kapillarspaltinnenraum und Nachweiselement und somit dem Anströmen des Nachweiselementes mit Flüssigkeit, während das Verteilen der Flüssigkeit innerhalb des Nachweiselementes dann durch spezielle Strukturen des Nachweiselements selbst erfolgt. Solche Anordnungen können insbesondere dann vorteilhaft sein, wenn Testelemente in großtechnischem Maßstab hergestellt werden, da durch die Verwendung gleich breiter Netzwerkstrukturen und Nachweiselemente die Produktionsverfahren erheblich vereinfacht werden können.

Überraschenderweise hat sich gezeigt, dass das erfindungsgemäße Testelement besonders geeignet ist, mehrere Parameter auf einem einzigen Testelement zu bestimmen. Hierzu enthält das Testelement nicht nur ein Nachweiselement, sondern mehrere Nachweiselemente, welche vorzugsweise unterschiedliche Analyten nachweisen können. Zur Erhöhung von Sensitivität oder Spezifität der Analytbestimmung oder Vergrößerung des detektierbaren Konzentrationsbereichs können jedoch auch mehrere Nachweiselement auf dem Testelement enthalten sein, welche den gleichen Analyten nachweisen. Hierbei können identische oder unterschiedliche Nachweisreaktionen eingesetzt werden.

Wird ein Testelement in Form eines Multi-Parameter-Teststreifens ausgeführt, befinden sich die Nachweiselemente vorzugsweise in Flussrichtung der Flüssigkeit hintereinander angeordnet.

Wie bereits erwähnt, erfolgt bei Testelementen, welche mit Vliesen oder ähnlichen Materialien zum Transport und zur Verteilung der Probenflüssigkeit arbeiten, der Flüssigkeitstransport in diesen Stoffen relativ langsam, so dass es hier zu deutlichen Verzögerungen bei der Anströmung der einzelnen Testelemente mit der Probenflüssigkeit und damit beim Beginn der Nachweisreaktion in den einzelnen Nachweiselementen kommt. Dies wiederum reduziert deutlich die Reproduzierbarkeit des Nachweises. Durch den langsamen Transport der Flüssigkeit an mehreren Nachweiselementen vorbei kann es weiterhin zu Verschleppungs- oder Verarmungs-Artefakten und somit zu ungenauen Analytbestimmungen kommen. Weiterhin treten in solchen Testelementen oft Dosierungsprobleme auf, da ein großer Teil der Flüssigkeitsprobe im Vlies selbst gebunden bleibt. Wird zu wenig Probenflüssigkeit aufgetragen, können die hinteren Nachweiselemente nicht mehr genügend Probenflüssigkeit für eine exakte Analytbestimmung erhalten. Wird hingegen zuviel Probenflüssigkeit aufgegeben, kann es zu einer Überdosierung der Probenmenge insbesondere bei den ersten Testelementen kommen. Weiterhin treten die zuvor erwähnten Nachteile größerer Probenmengen für den Patienten ein.

Überraschenderweise können durch die erfindungsgemäße Verwendung wie in Anspruch 10 definiert eines Netzwerkes, welches einen Kapillarspalt begrenzt, diese Probleme bisheriger Multi-Parameter-Testelemente vermieden werden.

Durch den erfindungsgemäßen Kapillarspalt erfolgt der Transport und die Verteilung der Flüssigkeitsmenge über die gesamte Länge des Kapillarspaltes innerhalb sehr kurzer Zeit, so dass jedes einzelne Nachweiselement weitgehend zeitgleich von der Flüssigkeitsprobe angeströmt werden kann. Hiermit ist ein weitgehend zeitgleicher und genau definierter Beginn der Nachweisreaktion und somit eine Erhöhung der Reproduzierbarkeit und Messgenauigkeit gegeben. Zusätzlich werden Verschleppungsprobleme vermieden. Weiterhin ist das Netzwerk selbst nicht kapillaraktiv, so dass der Flüssigkeitstransport aus dem Kapillarspalt nur in den Bereichen der Nachweiselemente erfolgt. Dies und die Tatsache, dass das Netzwerk selbst kein kapillaraktives Eigenvolumen ausweist, minimieren die benötigte Probenmenge deutlich, so dass eine geringere Beeinträchtigung des Patienten auftritt.

In einer besonders bevorzugten Ausführungsform sind mehrere Nachweiselemente hintereinander auf dem Netzwerk angeordnet und jeweils durch Bereiche, welche der Befestigung und/oder Abtrennung der einzelnen Nachweiselemente voneinander dienen, getrennt. Diese Befestigungs- und Trennbereiche besitzen bevorzugt flüssigkeitsabweisende, insbesondere hydrophobe, Eigenschaften und grenzen direkt an die jeweiligen Nachweiselemente an. Hierdurch schränken sie den Transport der Flüssigkeit in der Form ein, dass lediglich ein Flüssigkeitstransport aus dem Kapillarspalt durch das Netzwerk hindurch in die Nachweiselemente möglich ist, ohne dass die Flüssigkeit in die Zwischenräume zwischen den einzelnen Nachweiselemente gelangen kann. Hierdurch kann sowohl die Verschleppungsproblematik als auch das benötigte Probenvolumen nochmals deutlich reduziert werden.

Überraschenderweise hat sich gezeigt, dass die Verwendung von Schmelzkleber zur Befestigung der einzelnen Nachweiselemente auch zur Abtrennung der einzelnen Nachweiselemente gegeneinander dienen kann. Hierzu werden die einzelnen Nachweiselemente zunächst auf das Netzwerk aufgebracht. In einem nachfolgendem Schritt werden die einzelnen Nachweiselemente vorzugsweise an den senkrecht zum Kapillarspalt stehenden Seiten der Nachweiselemente in einem Fachmann bekannter Weise mit Schmelzkleber fixiert, so dass der Schmelzkleber in flüssiger Form auf die Kanten des jeweiligen Nachweiselemente aufgebracht wird und in das darunter liegende Netzwerk einfließt und erkaltet. Hierdurch sind einerseits die Nachweiselemente selbst in direktem Kontakt zum Netzwerk fixiert, andererseits durch den hydrophoben Schmelzkleber in den Zwischenräumen voneinander getrennt. Eigenschaften und Verarbeitungsbedingungen des Schmelzklebers wie Viskosität, Schmelztemperatur oder Abkühlrate können von einem Fachmann derartig gewählt werden, dass der Schmelzkleber in das Netzwerk einfließt und die Nachweiselemente an diesem fixiert und gegeneinander abtrennt, aber nicht durch das Netzwerk in den Kapillarspalt selbst einfließt, wodurch der Transport der Flüssigkeit innerhalb des Kapillarspaltes stark beeinflusst oder sogar ganz unterbunden wäre.

Besonders bevorzugt kann ein solches Multi-Parameter-Testelement zum Nachweis von Analyten eingesetzt werden, welche in einem diagnostischen Zusammenhang stehen. So können beispielsweise mehrere verschiedene Analyten auf einem gemeinsamen Testelement bestimmt werden, von denen bekannt ist, dass ihre Konzentrationen oder ihr generelles Fehlen oder Vorhandensein bei Vorliegen eines bestimmten Krankheitsbildes jeweils in charakteristischer Weise verändert sind. Durch das gleichzeitige Messen mehrerer solcher zusammenhängender Parameter können zeitgleich und mit einem einzigen Arbeitsgang verschiedene Analyten bestimmt werden, was eine rasche diagnostische Aussage ermöglicht. Weiterhin können durch Berücksichtigung von spezifischen Kombinationen der einzelnen Analyseergebnisse oft diagnostische Aussagen getroffen werden, welche durch die Betrachtung nur eines Parameters nicht möglich sind. Insbesondere kann so die Spezifität und/oder Sensitivität des diagnostischen Verfahrens gesteigert werden. Solche Multi-Parameter-Testelemente können beispielsweise als Lipid-Panel mit Nachweiselementen für Gesamt-Cholesterin, HDL-Cholesterin, LDL-Cholesterin und/oder Triglyzeride ausgeführt sein. Weitere mögliche Multi-Parameter-Testelemente können beispielsweise die vorgenannten Lipidparameter und weitere Parameter wie beispielsweise Glukose beinhalten. Auch Testelemente zur simultanen Bestimmung von Glukose und glykosyliertem Hämoglobin und/oder Gesamthämoglobin sind möglich.

Insbesondere kann ein erfindungsgemäßes Multi-Parameter-Testelement zur Bestimmung von Parametern eingesetzt werden, welche in Zusammenhang mit einem erhöhten Risiko oder einem Vorliegen von Herz-Kreislauf-Erkrankungen stehen. Derartige Parameter sind insbesondere Gesamt-Cholesterin, HDL-Cholesterin, LDL-Cholesterin oder Triglyzeride. Hierbei werden die jeweiligen Nachweiselemente hintereinander auf dem Netzwerk in vorgenannter Weise fixiert. Besonders bevorzugt ist eine Kombination von Nachweiselementen für Cholesterin, HDL-Cholesterin und Triglyzeride auf einem Testelement. Die Nachweiselemente hierfür können in dem Fachmann bekannter Weise hergestellt werden. Insbesondere können sie im vorliegenden Fall auf Nachweisverfahren beruhen, welche für reflektorische Analytbestimmungen eingesetzt werden. Solche Nachweisverfahren sind beispielsweise in den Datenblättern zu Reflotron® HDL Cholesterol, Reflotron® Cholesterol und Reflotron® Triglycerides (alle von Roche Diagnostics, Mannheim, Deutschland) beschrieben.

Die oben genannte Aspekte der Erfindung können entweder alleine oder in beliebiger Kombination verwendet werden.

Ein weiterer Gegenstand der Erfindung ist die Verwendung eines erfindungsgemäßen analytischen Testelements zur Bestimmung eines Analyten in einer Flüssigkeit.

Das erfindungsgemäße analytische Testelement, wie in einem der Ansprüche 1 bis 9 definiert, kann in einem Verfahren zur Bestimmung eines Analyten in einer flüssigen Probe, insbesondere einer Körperflüssigkeit wie Blut, Plasma, Serum, Urin, Speichel, Schweiß etc., eingesetzt werden. Insbesondere handelt es sich um ein Verfahren zur Bestimmung eines Analyten in einer Flüssigkeit mit Hilfe eines erfindungsgemäßen analytischen Testelements, wobei die Flüssigkeit mit dem Testelement, bevorzugt in einem speziell ausgeformten Probenaufgabebereich, in Kontakt gebracht wird, die Flüssigkeit durch Kapillarkräfte in dem zum Flüssigkeitstransport befähigten Kanal mindestens bis zum Bereich des Nachweiselementes transportiert wird, im Bereich des Nachweiselementes durch das Netzwerk hindurch zum Nachweiselement transportiert wird und dort mit den im Nachweiselement enthaltenen Reagenzien eine analytspezifische, insbesondere visuell oder apparativ-optisch, bevorzugt reflexionsphotometrisch beobachtbare, Nachweisreaktion eingeht, wodurch auf die Anwesenheit und gegebenenfalls auf die Menge des zu bestimmenden Analyten geschlossen werden kann.

Dabei wird zunächst die flüssige Probe, bevorzugt an einer speziell ausgeformten Probenaufgabeöffnung, mit dem Testelement kontaktiert. Durch Kapillarkräfte wird die Probenflüssigkeit in den zum kapillaren Flüssigkeitstransport befähigten Kanal transportiert. Der Transport erfolgt hierbei zumindest bis zu den Bereichen des Kapillarspaltes, welchen dem Nachweiselement gegenüberliegen. Die Flüssigkeitsprobe kann in diesen Bereichen über das Netzwerk hinweg in das Nachweiselement eindringen und mit den im Nachweiselement enthaltenen Reagenzien eine analytspezifische, insbesondere visuell oder apparativ-optisch, bevorzugt reflexionsphotometrisch beobachtbare, Nachweisreaktion eingehen. Anhand dieser kann auf die An- bzw. Abwesenheit und gegebenenfalls die Menge des zu bestimmenden Analyten geschlossen werden.

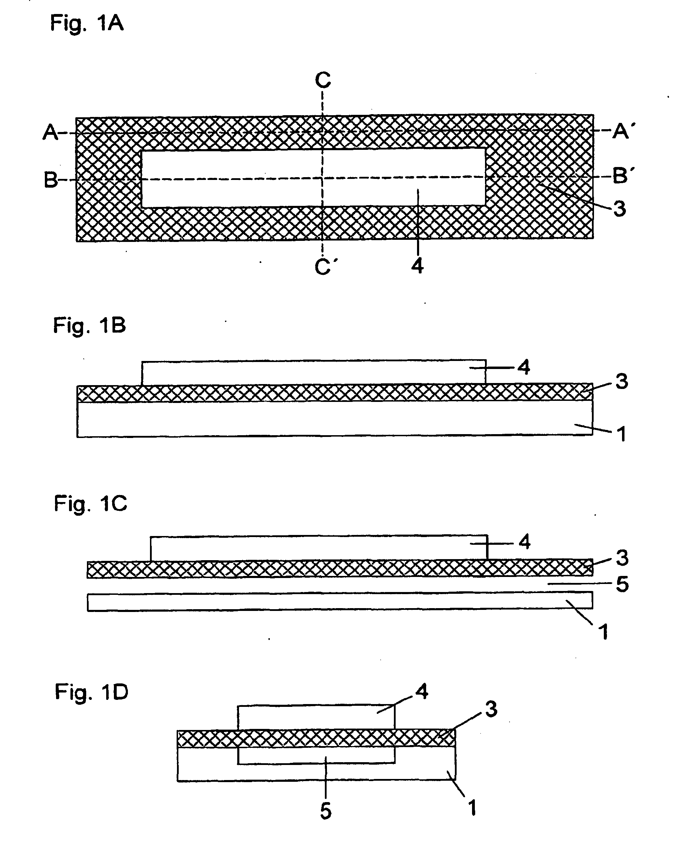

Die Erfindung wird durch die beigefügten Figuren und Beispiele weiter erläutert, wobei die Figuren 1 bis 4 bevorzugte Ausführungsformen des erfindungsgemäßen Testelements zeigen.

Figur 1 zeigt eine besonders bevorzugte Ausführungsform eines erfindungsgemäßen Testelements. In Figur 1A ist eine schematische Aufsicht auf das erfindungsgemäße Testelement zu sehen, die Figuren 1B bis 1D zeigen jeweils Querschnittsdarstellungen entlang der Linien A-A', B-B' beziehungsweise C-C'.

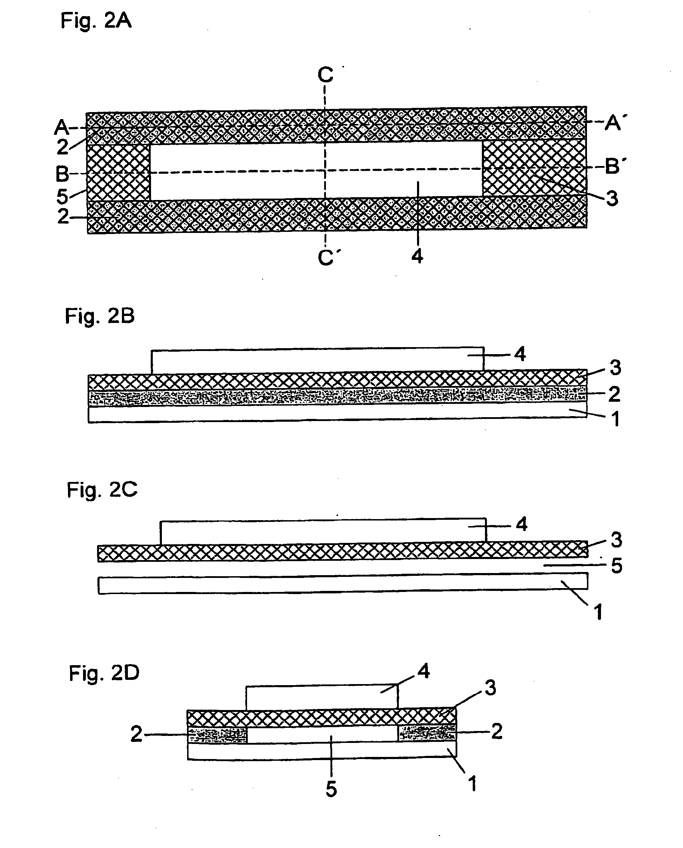

Figur 2 zeigt eine besonders bevorzugte Ausführungsform eines erfindungsgemäßen Testelements. In Figur 2A ist eine schematische Aufsicht auf das erfindungsgemäße Testelement zu sehen, die Figuren 2B bis 2D zeigen jeweils Querschnittsdarstellungen entlang der Linien A-A', B-B' beziehungsweise C-C'.

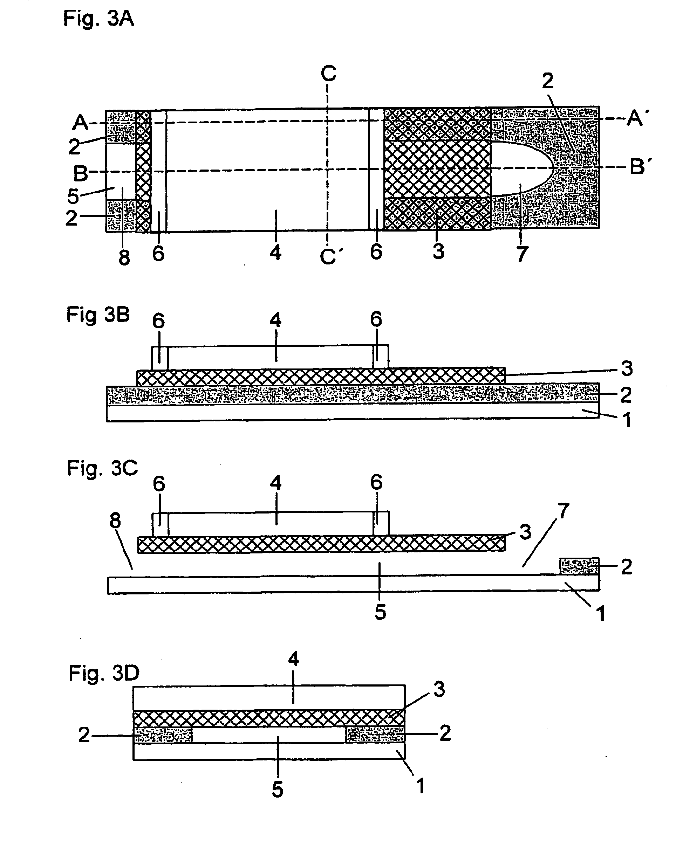

Figur 3 zeigt eine weitere besonders bevorzugte Ausführungsform eines erfindungsgemäßen Testelements mit speziell ausgeformten Probenaufgabebereich und Entlüftungsöffnung. In Figur 3A ist eine schematische Aufsicht auf das erfindungsgemäße Testelement abgebildet. Die Figuren 3B bis 3D zeigen jeweils Querschnitte entlang der Linien A-A', B-B' beziehungsweise C-C'.

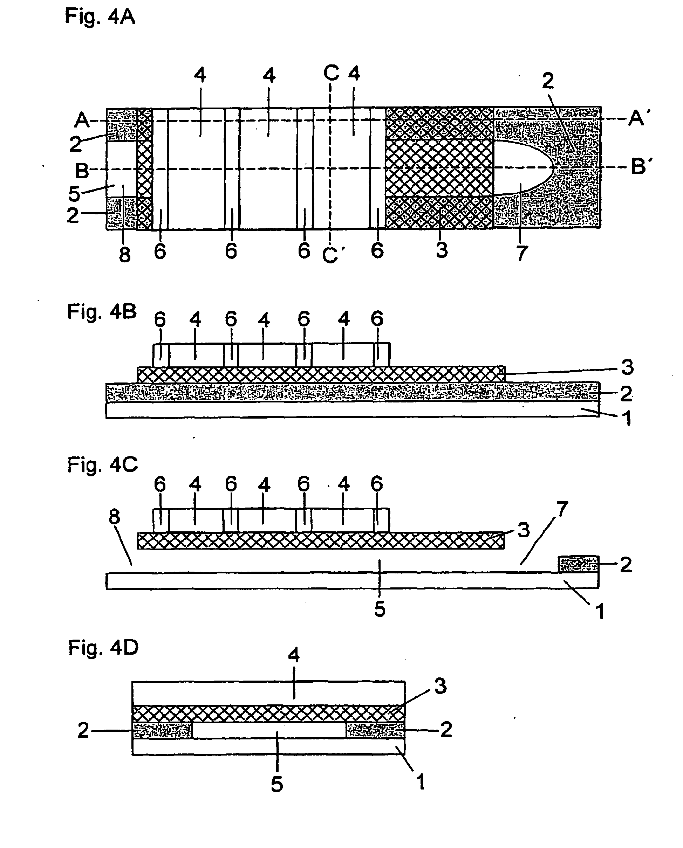

Figur 4 zeigt ebenfalls eine besonders bevorzugte Ausführungsform eines erfindungsgemäßen Multi-Parameter-Testträgers mit mehreren hintereinanderliegenden Nachweiselementen und speziell ausgeformten Probenaufgabebereich und Entlüftungsöffnung. Figur 4A ist eine Aufsicht auf das Testelement. Die Figuren 4B bis 4D zeigen jeweils Querschnitte entlang der Achsen A-A', B-B' beziehungsweise C-C'.

Die Ziffern in den Figuren bedeuten:
- 1: Träger
- 2: Zwischenschicht
- 3: Netzwerk
- 4: Nachweiselement
- 5: Kapillarer Kanal
- 6: Befestigungselemente
- 7: Probenaufgabebereich
- 8: Entlüftungsöffnung

### Beschreibung der Figuren

In Figur 1 ist eine besonders bevorzugte Ausführungsform des erfindungsgemäßen Testelements schematisch in verschiedenen Ansichten dargestellt (Figur 1A bis 1D). Die gezeigten Ansichten sollen in ihrer Kombination einen plastischen Eindruck des erfindungsgemäßen Testelements liefern. Figur 1A zeigt eine Aufsicht auf das Testelement, wobei der Träger (1) selbst nicht sichtbar ist, da er in dieser Ausführungsform vom Netzwerk (3) beziehungsweise dem Nachweiselement (4) komplett überdeckt ist. Das Testelement besteht aus einem Träger (1), der so geformt ist, dass er dort, wo er mit dem Netzwerk (3) bedeckt ist, zusammen mit diesem einen kapillaren Kanal (5) bildet. Beispielsweise kann eine Vertiefung in den Träger geprägt, geätzt oder gefräst sein. In der dargestellten Ausführungsform ist der Träger (1) U-förmig ausgebildet, wie in Figur 1D im Querschnitt entlang der Linie C-C' zu sehen ist.

Die Figuren 1B bzw. 1C stellen Längsschritte durch das Testelement dar, welche entlang den Linien A-A' bzw. B-B' laufen. Figur 1B zeigt einen Längsschnitt im Bereich der seitlichen Wände des kapillaren Kanals, Figur 1C einen Längsschnitt im Bereich des kapillaren Kanals.

Die Befestigung des Netzwerkes (3) am Träger (1) erfolgt vorzugsweise durch Verkleben, kann aber auch mit anderen dem Fachmann bekannten Techniken, wie z. B. Verschweißen, erfolgen. In der gezeigten Ausführungsform ist das Netzwerk (3) über der ganzen Grundfläche des Trägers (1) derartig aufgebracht, dass es zusammen mit diesem einen kapillaren Kanal bildet, welcher es ermöglicht, zur Probenaufgabe einen Flüssigkeitstropfen unmittelbar mit dem kapillaren Kanal (5) in Kontakt zu bringen. An der dieser Probenaufgabeseite gegenüberliegenden Seite des kapillaren Kanals befindet sich aufgrund der offenen Bauweise des Kanals eine Entlüftungsöffnung, welche bei der kapillaren Befüllung des Kanals mit Probenflüssigkeit das Entweichen von Luft und somit ein vollständiges und gleichmäßiges Befüllen des kapillaren Kanals erlaubt. Weiterhin sind Ausführungsformen des Testelements möglich, bei welchen die Entlüftung durch das Netzwerk selbst erfolgt, so dass auf eine spezielle Entlüftungsöffnung verzichtet werden kann. Hierbei ist das Netzwerk vorzugsweise so mit den anderen Bauteilen des Testelements in Verbindung zu setzen, dass eine Entlüftung hinter dem Nachweiselement, oder im Falle eines Multi-Parameter-Testelements hinter dem letzten Nachweiselement, gewährleistet ist, um eine optimale Befüllung zu gewährleisten. Der kapillare Kanal (5) reicht von der Probenaufgabestelle über den Bereich, welcher unterhalb des Nachweiselementes (4) liegt, bis zur Entlüftungsöffnung am entgegengesetzten Ende und gewährleistet somit eine homogene Probenverteilung insbesondere in dem Bereich, welcher unterhalb des Nachweiselements (4) liegt.

Das Nachweiselement (4) befindet sich in direktem Kontakt zum Netzwerk (3). Die Befestigung des Nachweiselementes (4) am Netzwerk (3) kann mit dem Fachmann bekannten Techniken erreicht werden. Allerdings ist hierbei darauf zu achten, dass ein Transport der Flüssigkeitsprobe von Netzwerk in das Nachweiselement möglich ist. Das Nachweiselement besitzt im vorliegenden Beispiel eine Breite, welcher der Breite des kapillaren Spalts entspricht und eine Länge, welche kürzer als die Länge des kapillaren Spaltes ist. In anderen Ausführungsformen sind auch Abmessungen des Nachweiselementes möglich, welche davon abweichen. Beispielsweise kann die Länge und/oder Breite des Nachweiselementes geringer sein als die entsprechenden Abmessungen des Kapillarspaltes, insbesondere in Fällen, bei welchen die Herstellung großflächiger Nachweiselemente aus finanziellen oder konstruktiven Gründen nicht sinnvoll erscheint. Andererseits kann die Länge und/oder Breite des Nachweiselementes größer sein als die entsprechenden Abmessungen des Kapillarspaltes, insbesondere in Fällen, bei welchen die Herstellung großflächiger Nachweiselemente kostengünstig ist und die entsprechenden Produktionsverfahren der Testelemente auf Nachweiselemente optimiert sind, welche die gleiche Breite wie das Netzwerk bzw. der Träger aufweisen. Dies ist insbesondere bei Teststreifen der Fall, welche erst nach ihrem Zusammensetzen der Einzelkomponenten in ihren endgültige Form unterteilt werden, beispielsweise durch Schneiden oder Stanzen flächiger Zwischenprodukte. In diesen Fällen besitzen die Nachweiselemente (4) bevorzugt spezielle Eigenschaften oder Strukturen, beispielsweise Vliesstrukturen, welche eine möglichst gleichmäßige Verteilung der Flüssigkeitsprobe im Nachweiselement ermöglichen.

Bei der Verwendung des gezeigten Testelements wird das Testelement mit der Probenaufgabestelle mit der Probenflüssigkeit, beispielsweise mit einem sich an der Fingerkuppe befindlichen Blutstropfen, in Kontakt gebracht. Dabei kommt die Probenflüssigkeit dem kapillaren Kanal (5) in Kontakt. Dieser füllt sich durch kapillare Kräfte solange mit der Probenflüssigkeit, bis er zumindest bis zum Bereich der Nachweiselemente gefüllt ist. Dies geschieht aufgrund der erfindungsgemäßen Konstruktion des Testelements in sehr kurzer Zeit, bevorzugt in wenigen Sekunden. Danach kann der Kontakt des Testelements zur restlichen Probenflüssigkeit, beispielsweise durch Entfernen des Testelements vom Patientenfinger, unterbrochen werden, da durch die erfindungsgemäße Funktionsweise des Testelements gewährleistet ist, dass in sehr kurzer Zeit die für die Bestimmung des Analyten erforderliche Probenmenge im Testelement vorliegt. Nach dem Befüllen des kapillaren Kanals (5) erfolgt aufgrund der erfindungsgemäßen Eigenschaften des Netzwerks und der durch die räumliche Anordnung von Netzwerk und Nachweiselement gemeinsam erzeugten kapillaren Zwischenräume der Transport der Flüssigkeitsprobe durch das Netzwerk (3) hindurch in das Nachweiselement (4). Dort erfolgt nun die Nachweisreaktion, mittels derer die An- oder Abwesenheit oder die Konzentration des zu bestimmenden Analyten ermittelt werden kann. Die zum Nachweis eines bestimmten Analyten möglichen Nachweisreaktion und Detektionsverfahren sind dem Fachmann bekannt und können von diesem entsprechend den erfindungsgemäßen Vorrichtungen und Verfahren eingesetzt werden.

In Figur 2 ist eine weitere besonders bevorzugte Ausführungsform als Alternative zu dem in Figur 1 dargestellten Testelement abgebildet. Die Teilansichten Figur 2A bis 2D sollen wiederum einen räumlichen Eindruck des erfindungsgemäßen Testelements vermitteln. Das gezeigte Testelement enthält erfindungsgemäß einen zum kapillaren Flüssigkeitstransport befähigten Kanal (5), der von einem inerten Träger (1), zwei Zwischenschichten (2) und dem Netzwerk (3) gebildet wird. Die beiden Zwischenschichten (2) bilden hierbei die seitlichen Begrenzungen, der Träger (1) die Bodenfläche und das Netzwerk (3) die Abdeckung des kapillaren Kanals (5). Besonders vorteilhaft zeigt sich bei dieser Ausführungsform, dass der Träger planar ausgebildet sein kann und keine zusätzlichen Herstellungs- oder Arbeitsschritte nötig sind, um eine Vertiefung in diesen einzubringen. Dies kann zu wesentlich einfacheren und kostengünstigeren Herstellverfahren solcher erfindungsgemäßer Testelemente führen. Die Geometrie des kapillaren Kanals kann hierbei durch die Abmessungen der Zwischenschichten bestimmt werden. So kann die Höhe des kapillaren Kanals, welche maßgeblich die kapillaren Eigenschaften bestimmt, durch die Schichtdicke der Zwischenschicht eingestellt werden. Durch den Abstand der beiden Zwischenschichten voneinander kann die Breite des kapillaren Kanals, welche maßgeblich dessen Querschnitt und damit dessen Volumen bestimmt, eingestellt werden. Dadurch kann das Volumen der Flüssigkeitsprobe im Kapillarspalt bestimmt werden, so dass mit Hilfe dieses geometrischen Parameters eine Volumendosierung zu einer möglichst exakten Analytbestimmung erfolgen kann. Durch die Länge der Zwischenschichten kann die Länge des kapillaren Kanals bestimmt werden, welche maßgeblich die Länge des Transportweges der Flüssigkeitsprobe vom Probenaufgabebereich zum Nachweiselement und damit den Abstand von Applikationsort und Detektionsort bestimmt, welcher wieder für eine möglichst einfache und hygienische Handhabung eine entscheidende Rolle spielt. Die geometrischen Parameter Länge, Breite und Höhe müssen hierbei nicht unbedingt die hier beschriebenen Funktionen erfüllen, sondern es ist auch möglich, dass einzelne dieser Parameter die Funktionen anderer Parameter übernehmen oder auch mehrere Funktionen gleichzeitig erfüllen können. So kann durch eine geeignete Wahl der Höhe der Zwischenschichten nicht nur dessen Kapillaraktivität beeinflusst werden, sondern auch noch das Volumen eingestellt und somit eine Dosierung der Flüssigkeitsprobe erzielt werden.

In einer besonders bevorzugten Ausführungsform sind die Zwischenschichten aus doppelseitig klebenden Bändern hergestellt. Diese ermöglichen einerseits über ihre geometrischen Abmessungen eine genaue geometrische Definition des kapillaren Kanals und ermöglichen gleichzeitig die räumliche Verbindung der einzelnen Bestandteile des kapillaren Spaltes. Hierzu werden vorzugsweise zunächst die beiden doppelseitig klebenden Bänder als Zwischenschichten (2) in einen genau definierten Abstand zueinander auf den Träger (1) geklebt. Anschließend wird, nach dem eventuellen Abziehen möglicher Schutzfolien vom Band, das Netzwerk (3) durch Aufbringen und Anpressen mit den doppelseitig klebenden Bändern in der Weise verklebt, dass ein kapillarer Kanal gebildet wird. Mit dieser besonders bevorzugten Ausführungsform ist eine sehr kostengünstige und einfache Herstellung solcher erfindungsgemäßer Testelemente möglich.

In Figur 3 wird schematisch anhand unterschiedlicher Ansichten (Figur 3A bis 3D) ist eine weitere besonders bevorzugte Ausführungsform eines erfindungsgemäßen Testelements abgebildet. Diese Ausführungsform weist eine Zwischenschicht (2) auf, welche in dieser speziellen Ausführungsform aus einem speziell doppelseitig klebendem Band besteht. Diese spezielle Ausformung dient insbesondere dazu, in Kombination mit dem an entsprechender Stelle aufgebrachten Netzwerk (3) einen Probenaufgabebereich (7) und eine Entlüftungsöffnung (8) auszubilden, welche ein problemloses und vollständiges Füllen des kapillaren Spaltes mit der Probenflüssigkeit gewährleisten. Die Ausgestaltung der Zwischenschicht (2) kann insbesondere durch Stanzen oder Schneiden erfolgen. Aufgrund des speziell gestalteten Probenaufgabebereichs (7) und der Entlüftungsöffnung (8) überdeckt das Netzwerk (3) in dieser Ausführungsform nicht unbedingt die gesamte Grundfläche des Trägers (1) oder der Zwischenschicht (2), sondern lässt insbesondere im Probenaufgabebereich einen Teil des Trägers bzw. der darauf angebrachten Zwischenschicht unbedeckt. Nicht von Netzwerk bedeckte Bereiche der Zwischenschicht können mit einer Abdeckung versehen werden. Dies ist insbesondere bei der Verwendung eines doppelseitig klebenden Bandes als Zwischenschicht vorteilhaft, da so die klebenden Bereiche außerhalb des Netzwerkes abgedeckt werden. Im Probenaufgabebereich wird nun zur Probenapplikation ein oder mehrere Tropfen der Probenflüssigkeit aufgegeben, welche aufgrund der speziellen Ausformung des Probenaufgabebereichs unverzüglich mit dem kapillaren Spalt (5) in Kontakt gelangen, so dass die Probenflüssigkeit unverzüglich mittels Kapillarkraft in den kapillaren Spalt transportiert wird und so unverzüglich zum Nachweiselement transportiert wird. Auch die Entlüftungsöffnung kann speziell ausgeformt sein. So kann es aus Gründen eines geringeren Materialverbrauchs sinnvoll sein, das Netzwerk und somit den kapillaren Kanal relativ unmittelbar nach dem Nachweiselement zu beenden, wie es in der dargestellten Ausführungsform dargestellt ist. Weiterhin überdeckt das Nachweiselement (4) in dieser bevorzugten Ausführungsform das Netzwerk (3) auch in Bereichen, welche nicht mehr direkt über dem kapillaren Kanal (5) liegen. Bevorzugt ist das Nachweiselement (4) in solchen Fällen derartig beschaffen, dass es Eigenschaften oder Strukturen aufweist, welche eine Verteilung der aus dem kapillaren Kanal anströmenden Flüssigkeit über das gesamte Nachweiselement bewirken können. So können insbesondere vliesartige Strukturen oder Papiere in Nachweiselement enthalten sein. Weiterhin wird das Nachweiselement (4) in dieser bevorzugten Ausführungsform von zwei Befestigungselementen (6) auf dem Netzwerk (3) fixiert. Diese Befestigungselemente sind zumindest an zwei Seiten des Nachweiselements angebracht und positionieren dieses in einer bestimmten Lage auf dem Netzwerk. Eine weitere Funktion dieser Befestigungselemente ist es, den Kontakt der Unterseite des Nachweiselements mit der Oberseite des Netzwerks in einer Weise zu ermöglichen, welche es erlaubt, durch kapillare Kräfte die Flüssigkeitsprobe vom kapillaren Kanal (5) über das Netzwerk (3) in das Nachweiselement (4) zu transportieren. Insbesondere kann dies durch Anpressen oder Andrücken des Nachweiselements an das Netzwerk erzielt werden. Hierzu können beispielsweise die Befestigungselemente derartig ausgestaltet sein, dass sie das Nachweiselement in den Randbereichen überdecken und somit an das Netzwerk fixieren. In einer besonders bevorzugten Ausführungsform erfolgt die Fixierung des Nachweiselementes auf dem Netzwerk durch das Aufbringen flüssigen Schmelzklebers an zwei Randbereichen des Nachweiselements und den benachbarten Bereichen des Netzwerks. Nach dem Erkalten fixiert der Schmelzkleber so einerseits das Nachweiselement in seiner Lage auf dem Netzwerk und andererseits bewirkt er über einen Anpressdruck in Richtung des Netzwerks, dass der zum Flüssigkeitsübertritt notwendige Kontakt zwischen Nachweiselement und Netzwerk entsteht.

Figur 4 zeigt eine besonders bevorzugte Ausführungsform eines erfindungsgemäßen Multi-Parameter-Testträgers mit mehreren hintereinander liegenden Nachweiselementen und speziell ausgeformten Probenaufgabebereich und Entlüftungsöffnung. Figur 4A ist eine Aufsicht auf das Testelement. Die Figuren 4B bis 4D zeigen jeweils Querschnitte entlang der Achsen A-A', B-B' beziehungsweise C-C'.

Das hier dargestellte Testelement weist exemplarisch drei Nachweiselemente (4) auf, welche durch Befestigungselemente (6) voneinander getrennt sind. Es sind aber auch Testelemente eingeschlossen, welche zwei oder mehr als drei Nachweiselemente besitzen. Die Nachweiselemente können hierbei den selben Analyten nachweisen, bevorzugt werden jedoch Nachweiselemente in Form eines Multi-Parameter-Testelements kombiniert, welche unterschiedliche Analyten nachweisen. Die Befestigungselemente besitzen bevorzugt flüssigkeitsabweisende, insbesondere hydrophobe, Eigenschaften und grenzen direkt an die Nachweiselemente an. Hierdurch schränken sie den Transport der Flüssigkeit in der Form ein, dass lediglich ein Flüssigkeitstransport aus dem Kapillarspalt durch das Netzwerk hindurch in das jeweilige Nachweiselement möglich ist, ohne dass es zu einem lateralen Vermischen der einzelnen Flüssigkeitssegmente und damit zu Verschleppungsproblemen kommen kann. Besonders bevorzugt kann ein solches Multi-Parameter-Testelement zum Nachweis von Analyten in Form eines Panel-Testes eingesetzt werden, welche in einem diagnostischen Zusammenhang stehen. Insbesondere kann ein erfindungsgemäßes Multi-Parameter-Testelement zur Bestimmung von Parametern eingesetzt werden, welche in Zusammenhang mit einem erhöhten Risiko oder einem Vorliegen von Herz-Kreislauf-Erkrankungen stehen. Derartige Parameter sind insbesondere Gesamt-Cholesterin, HDL-Cholesterin, LDL-Cholesterin oder Triglyzeride. Besonders bevorzugt ist eine Kombination von Nachweiselementen für Cholesterin, HDL-Cholesterin und Triglyzeride, welche in Form eines Panel-Testes hintereinander auf dem Testelement angeordnet sind.

### Beispiele:

### Beispiel 1:

### Herstellung eines erfindungsgemäßen analytischen Testelements (vgl. Fig. 4)

Auf eine 350 µm dicke Trägerfolie aus Polyethylenterephthalat (Melinex®, ICI, Frankfurt am Main, Deutschland), welche zuvor durch Behandlung mit Dioctylnatriumsulfosuccinat (2% in Ethanol, Merck KgaA, Darmstadt, Deutschland) hydrophilisiert wurde, wird einige mm von Rand entfernt ein doppelseitiges Klebeband der Dicke 200 µm geklebt. Die Trägerfolie besitzt eine Länge von 75 mm und Breite von 5 mm. Das Klebeband besitzt eine Länge von 35 mm und ebenfalls eine Breite von 5 mm. Weiterhin besitzt das Klebeband an dem Ende, welches der späteren Entlüftungsöffnung entspricht, eine mittige Ausstanzung von 2 mm Breite und 15 mm bis 25 mm Länge, welche die Dimensionen des Kapillarkanals definiert. Auf der der Entlüftungsöffnung gegenüberliegenden Seite der Ausstanzung schließt sich vorzugsweise ein speziell ausgeformter Probenaufgabebereich an. Dieser wird durch eine ovale Ausstanzung von 6 mm Länge und 3 mm Breite gebildet. Die Länge der Ausstanzung kann geringfügig größer als die gewünschte Länge des kapillaraktiven Kanals, die durch dessen Abdeckung bestimmt wird, gewählt werden, um eine Entlüftung des Kanals während des Befüllens mit Probenflüssigkeit zu gewährleisten. Auf das Klebeband wird über die Länge des Kapillarspaltes, d.h. vom Probenaufgabebereich bis zur Entlüftungsöffnung, ein Netzwerk der Breite 5 mm geklebt. Das Netzwerk besteht aus monofilem PET-Gewebe (Sefar Petex 07-98/34 der Sefar AG, Rueschlikon, Schweiz). Zur Verbesserung der erfindungsgemäßen Transporteigenschaften für Flüssigkeiten wird das Netzwerk zusätzlich durch Tränken mit 0,1% Dioctylnatriumsulfosuccinat (Merck KgaA, Darmstadt, Deutschland) hydrophilisiert. Die Bereiche des ausgestanzten Klebebands, welche im Bereich der Probenaufgabestelle nicht vom Netzwerk überdeckt sind, werden zusätzlich mit einer inerten Abdeckfolie überdeckt. Auf dem Netzwerk, welches über das Klebeband mit der Trägerfolie verbunden ist, werden 3 Nachweiselemente mittels Schmelzkleber fixiert, so dass der Flüssigkeitstransport vom Kapillarkanal durch das Netzwerk hindurch zu den Nachweiselementen möglich ist. Hierzu wird der Schmelzkleber an den senkrecht zum Kapillarkanal stehenden Seiten der Nachweiselemente in noch flüssiger Form aufgebracht und fließt zumindest teilweise in das Netzwerk ein. Nach dem Erstarren des Schmelzklebers sind die Nachweiselemente in einer Weise an das Netzwerk fixiert, welche das seitliche Verschieben der Nachweiselemente verhindert. Weiterhin werden die Nachweiselemente durch die Fixierung mit Schmelzkleber an das Netzwerk angedrückt, so dass die Flüssigkeitsprobe vom Kapillarkanal über das Netzwerk in das Nachweiselement einströmen kann. Hierbei ist besonders darauf zu achten, dass kein Schmelzkleber durch das Netzwerk in den Kapillarspalt einfließt, da dadurch der Transport der Probenflüssigkeit im Kapillarspalt beeinflusst oder sogar vollständig unterbunden werden kann. Die Nachweiselemente enthalten für die Nachweisreaktionen des jeweiligen Analyten notwendigen Reagenzien sowie gegebenenfalls Hilfsstoffe. Solch ein Testelement ist insbesondere als Multi-Parameter-Testelement zur Bestimmung von Parametern geeignet, welche in Zusammenhang mit einem erhöhten Risiko oder einem Vorliegen von Herz-Kreislauf-Erkrankungen stehen. Hierzu werden Nachweiselemente für HDL-Cholesterin, Cholesterin und Triglyzeride hintereinander auf dem Netzwerk mit Schmelzkleber fixiert. Solche Nachweiselemente sind beispielsweise in den Datenblättern zu Reflotron® HDL Cholesterol, Reflotron® Cholesterol und Reflotron® Triglycerides (alle von Roche Diagnostics, Mannheim, Deutschland) beschrieben. Der Schmelzkleber dient hierbei nicht ausschließlich der Fixierung der Nachweiselemente, sondern bewirkt auch, dass keine Flüssigkeit von einem Nachweiselement zum benachbarten gelangt und minimiert somit Verschleppungsprobleme. Hierbei können die Nachweiselemente beispielsweise eine Breite von 5 mm und eine Länge von ca. 4 mm aufweisen. Die Stege aus Schmelzkleber zur Fixierung und Abtrennung der einzelnen Nachweiselemente besitzen bevorzugt eine Breite von 5 mm und eine Länge von 1 bis 2 mm.

### Beispiel 2:

### Messung von Lipid-Parametern mit Hilfe des Testelements aus Beispiel 1

Das Testelement aus Beispiel 1 wird im Probenaufgabebereich mit einem Tropfen Blut in Kontakt gebracht. Die Kapillare des Testelements füllt sich innerhalb von 3-5 Sekunden selbständig und gleichmäßig mit der Probenflüssigkeit. In den Bereichen der Nachweiselemente strömt die Probenflüssigkeit über das Netzwerk weitgehend simultan in diese ein, wodurch die jeweilige Nachweisreaktion gestartet wird. Innerhalb weniger Sekunden wird eine Farbentwicklung im Nachweiselement sichtbar, welche nach Ablauf der Messzeit zur Bestimmung des Analyten herangezogen wird. Diese Messzeit beträgt beispielsweise für eine HDL Cholesterol-Bestimmung nach dem Prinzip Reflotron® HDL Cholesterol ca. 135 sec, für eine Cholesterol-Bestimmung nach dem Prinzip Reflotron® Cholesterol ca. 135 sec und für eine Triglyzerid-Bestimmung nach dem Prinzip Reflotron® HDL Triglycerides ca. 180 sec. Die Intensität der Färbung des Nachweiselementes wird reflexionsphotometrisch bestimmt. Diese Intensität der Färbung wird mit der Konzentration des Analyten in der Probe korreliert.

## Patentansprüche

1. Analytisches Testelement zur Bestimmung mindestens eines Analyten in einer Flüssigkeit, enthaltend einen Träger, mindestens ein Nachweiselement und einen zum kapillaren Flüssigkeitstransport befähigten Kanal,
dadurch gekennzeichet, dass der zum kapillaren Flüssigkeitstransport befähigte Kanal zumindest teilweise von einen hydrophilen Netzwerk gebildet wird, welches zumindest teilweise auf der einen Seite mit dem Innenraum des Kanals und auf der gegenüberliegenden Seite zumindest teilweise mit dem Nachweiselement in direktem Kontakt steht,
wobei das Netzwerk ein monofiles Gewebe ist und eine Maschenweite von 10 bis 500 µm, bevorzugt von 20 bis 300 µm, besonders bevorzugt von 50 bis 150 µm, besitzt sowie eine Dicke von 10 bis 500 µm, bevorzugt von 20 bis 300 µm, besonders bevorzugt von 50 bis 150 µm, besitzt,
so dass ein Flüssigkeitstransport vom Kanal über das Netzwerk zum Nachweiselement möglich ist.

2. Analytisches Testelement gemäß Anspruch 1,
**dadurch gekennzeichnet, dass** das Netzwerk selbst nicht saugfähig ist und beim senkrechten Eintauchen in Wasser eine Steighöhe des Wassers im Netzwerk von weniger als 2 mm ermöglicht.

3. Analytisches Testelement gemäß einem der Ansprüche 1 oder 2,
**dadurch gekennzeichnet, dass** der zum kapillaren Flüssigkeitstransport befähigte Kanal einen Probenaufgabebereich an einem und/oder eine Entlüftungsöffnung am gegenüberliegenden Ende des Kanals besitzt.

4. Analytisches Testelement gemäß einem der Ansprüche 1 bis 3,
**dadurch gekennzeichnet, dass** neben dem Netzwerk zumindest eine weitere der dem Innenraum des zum kapillaren Flüssigkeitstransports befähigten Kanals zugewandten Oberflächen hydrophil ist.

5. Analytisches Testelement gemäß einem der Ansprüche 1 bis 4,
**dadurch gekennzeichnet, dass** das Netzwerk aus Polyethylenterephthalat besteht, welches vorzugsweise durch eine Behandlung mit einem Netzmittel hydrophilisiert ist.

6. Analytisches Testelement gemäß einem der Ansprüche 1 bis 5,
**dadurch gekennzeichnet, dass** das monofile Gewebe einen Faserdurchmesser von 10 bis 300 µm, bevorzugt von 30 bis 150 µm, besonders bevorzugt von 50 bis 100 µm besitzt

7. Analytisches Testelement gemäß einem der Ansprüche 1 bis 6,
**dadurch gekennzeichnet, dass** das Nachweiselement die für die Nachweisreaktion des Analyten in der Flüssigkeit notwendigen Reagenzien sowie gegebenenfalls weitere Hilfsstoffe oder Hilfsstrukturen, insbesondere Filterstrukturen für partikuläre Probenbestandteile, enthält.

8. Analytisches Testelement gemäß einem der Ansprüche 1 bis 7,
**dadurch gekennzeichnet, dass** mehrere Nachweiselemente auf der dem Kanal abgewanden Seite des Netzwerkes angeordnet sind.

9. Analytisches Testelement gemäß einem der Ansprüche 1 bis 8,
**dadurch gekennzeichnet, dass** das oder die Nachweiselemente die Anwesenheit und gegebenenfalls die Konzentration von Cholesterol, HDL Cholesterol oder Triglyzeriden oder einer Kombination dieser Stoffe nachweisen können.

10. Verwendung eines analytischen Testelements gemäß einem der Ansprüche 1 bis 9 zur Bestimmung eines Analyten in einer Flüssigkeit.

## Claims

1. Analytical test element for determining at least one analyte in a liquid comprising a support, at least one detection element and a channel capable of capillary liquid transport
**characterized in that**
the channel capable of capillary liquid transport is at least partially formed by a hydrophilic network, one side of which is at least partially in direct contact with the inner space of the channel and the opposite side of which is at least partially in direct contact with the detection element,
wherein the network is a monofilament fabric and has a mesh width of 10 to 500 µm, preferably of 20 to 300 µm, particularly preferably of 50 to 150 µm and has a thickness of 10 to 500 µm, preferably of 20 to 300 µm, particularly preferably of 50 to 150 µm,
such that liquid can be transported from the channel across the network to the detection element.

2. Analytical test element according to claim 1,
**characterized in that**
the network itself is not absorbent and enables water to rise by less than 2 mm in the network when it is immersed vertically in water.

3. Analytical test element according to one of the claims 1 or 2,
**characterized in that**
the channel capable of capillary liquid transport has a sample application area at one end and/or a vent hole at the opposite end of the channel.

4. Analytical test element according to one of the claims 1 to 3,
**characterized in that**
in addition to the network at least one further surface facing the inner space of the channel capable of capillary liquid transport is hydrophilic.

5. Analytical test element according to one of the claims 1 to 4,
**characterized in that**
the network consists of polyethylene terephthalate which is preferably hydrophilized by treatment with a wetting agent.

6. Analytical test element according to one of the claims 1 to 5,
**characterized in that**
the monofilament fabric has a fibre diameter of 10 to 300 µm, preferably of 30 to 150 µm, particularly preferably of 50 to 100 µm.

7. Analytical test element according to one of the claims 1 to 6,
**characterized in that**
the detection element contains the reagents necessary for the detection reaction of the analyte in the liquid and optionally further auxiliary substances or auxiliary structures, in particular filter structures for particulate sample components.

8. Analytical test element according to one of the claims 1 to 7,
**characterized in that**
several detection elements are arranged on the side of the network facing away from the channel.

9. Analytical test element according to one of the claims 1 to 8,
**characterized in that**
the detection element or the detection elements can detect the presence and optionally the concentration of cholesterol, HDL cholesterol or triglycerides or a combination of these substances.

10. Use of an analytical test element according to one of the claims 1 to 9 for determining an analyte in a liquid.

## Revendications

1. Élément de test analytique pour déterminer au moins un analyte dans un liquide, comprenant un support, au moins un élément de détection et un canal apte au transport capillaire de liquide,
**caractérisé en ce que** le canal apte au transport capillaire de liquide est formé au moins partiellement par un maillage hydrophile qui est en contact direct, au moins partiellement, sur un côté avec l'intérieur du canal et sur le côté opposé, au moins partiellement, avec l'élément de détection,
ledit maillage étant un tissu monofil et ayant une largeur de maille de 10 à 500 µm, de préférence de 20 à 300 µm, de manière particulièrement préférée de 50 à 150 µm, ainsi qu'une épaisseur de 10 à 500 µm, de préférence de 20 à 300 µm, de manière particulièrement préférée de 50 à 150 µm,
permettant ainsi un transport de liquide du canal via le maillage vers l'élément de détection.

2. Élément de test analytique selon la revendication 1,
**caractérisé en ce que** le maillage lui-même n'est pas absorbant et permet en immersion verticale dans l'eau une hauteur de remontée de l'eau dans le maillage de moins de 2 mm.

3. Élément de test analytique selon l'une des revendications 1 ou 2,
**caractérisé en ce que** le canal apte au transport capillaire de liquide comporte une zone de dépôt d'échantillon à une extrémité du canal et/ou un orifice d'évacuation d'air à l'extrémité opposée du canal.

4. Élément de test analytique selon l'une des revendications 1 à 3, **caractérisé en ce que**, outre le maillage, au moins une autre des surfaces tournées vers le canal apte au transport capillaire de liquide est hydrophile.

5. Élément de test analytique selon l'une des revendications 1 à 4, **caractérisé en ce que** le maillage est en polyéthylène téréphtalate, de préférence rendu hydrophile par un traitement avec un agent mouillant.

6. Élément de test analytique selon l'une des revendications 1 à 5, **caractérisé en ce que** le tissu monofil a un diamètre de fibre de 10 à 300 µm, de préférence de 30 à 150 µm, de manière particulièrement préférée de 50 à 100 µm.

7. Élément de test analytique selon l'une des revendications 1 à 6, **caractérisé en ce que** l'élément de détection comporte les réactifs nécessaires pour la réaction de détection de l'analyte dans le liquide ainsi que le cas échéant d'autres agents auxiliaires ou structures auxiliaires, en particulier des structures filtrantes pour des composants d'échantillon particulaires.

8. Élément de test analytique selon l'une des revendications 1 à 7, **caractérisé en ce que** plusieurs éléments de détection sont placés sur le côté opposé au canal du maillage.

9. Élément de test analytique selon l'une des revendications 1 à 8, **caractérisé en ce que** le ou les éléments de détection sont capables de détecter la présence et le cas échéant la concentration de cholestérol HDL ou de triglycérides ou d'une combinaison de ces substances.

10. Utilisation d'un élément de test analytique selon l'une des revendications 1 à 9 pour déterminer un analyte dans un liquide.
